# EUROPEAN PATENT SPECIFICATION

(11) **EP 0 461 141 B1**
(45) Date of publication and mention of the grant of the patent: **03.11.1999**
(21) Application number: 90903699.8
(22) Date of filing: 23.02.1990
(51) Int. Cl.: C07D 493/04, A61K 31/36, A61K 31/44, A61K 31/47

(54) **ETOPOSIDE ANALOGUES**
ETOPOSIDANALOGE
ANALOGUES D'ETOPOSIDE

(30) Priority: 23.02.1989 US 313826; 12.09.1989 US 406330
(43) Date of publication of application: 18.12.1991
(73) Proprietor: UNIVERSITY OF NORTH CAROLINA AT CHAPEL HILL, Chapel Hill, North Carolina 27514 (US)
(72) Inventor: LEE, Kuo-Hsiung, Chapel Hill, NC 27514 (US); WANG, Zhe, Qing, Chapel Hill, NC 27514 (US); CHENG, Yung-Chi, Woodbridge, CT 06525 (US); LIU, Su-Ying, Belmont, CA 94002 (US); IMAKURA, Yasuhiro, Tokushima 770 (JP); HARUNA, Mitsumasa 1-1729, Shirotauchi, Aichi-gun Aichi 470-01 (JP); BEERS, Scott, A., New Jersey 08887 (US); THURSTON, Lee, S., Virginia Beach, VA 23456 (US); DAI, Hua-Juan Room 302, A-14, Shangai (CN); CHEN, Chung-Hsiung, Taipei (TW); BOWEN, Phillip, J., Chapel Hill, NC 27514 (US); SCHNUR, Dora, M., St. Louis, MO 63132 (US); KUO, Yao, Haur Chinese Culture University, Taipei (TW); MORI, Masami 46-2, Inui Higaghiazai, Aichi-ken 491-01 (JP)
(74) Representative: Raynor, John
(86) International application number: US9000842
(87) International publication number: WO9009788

(56) References cited:
- FR-A- 1 502 813
- JP-A- 0 193 589
- JP-A- 1 117 885
- JP-A- 6 310 789
- JP-A- 6 323 884
- US-A- 2 977 359
- US-A- 2 984 674
- US-A- 3 524 844
- US-A- 4 122 092
- US-A- 4 144 336
- US-A- 4 567 253
- US-A- 4 788 216
- US-A- 4 808 592
- JOURNAL OF NATURAL PRODUCTS, vol. 52, no. 3, May-June 1989, pages 606-613, Columbus, Ohio, US; K.-H. LEE et al.: "Antitumor agents, 107. New cytotoxic 4-alkylamino analogues of 4'-demethyl-epipodophyllotoxin as inhibitors of human DNA topoisomerase II"
- ANTI-CANCER DRUG DESIGN, vol. 2, no. 3, 1987, pages 247-252, Basingstoke, GB; A.R. BEARD et al.: "Podophyllotoxin analogues: synthesis and computer modelling investigation of structure-activity relationships"
- JOURNAL OF THE CHEMICAL SOCIETY PERKIN TRANSACTIONS I, no. 8, 1976, pages 832-836, London, GB; D.C. AYRES et al.: "Lignans and related phenols. Part XV. Remote substituent effects on the rates and products of some reactions of aryltetrahydronaphthalenes"
- JOURNAL OF THE CHEMICAL SOCIETY, PERKIN TRANSACTIONS I, no. 11, 1972, pages 1350-1355, London, GB; C.D. AYRES et al.: "Lignans and related phenols. Part XIII. Halogenated derivatives of podophyllotoxin"
- CHEMICAL ABSTRACTS, Vol. 105, (THURSTON et al) entry 60444U, 1986.
- Helvetica Climica Acta, Vol. 52, No. 4, 1969, (KUHN et al) Article No. 106, pp 944-947.

## Description

This invention relates to compounds that are analogs of etoposide having antitumor activity. This invention also relates to a method for treating tumors by administering a safe and effective amount of the etoposide analog compounds.

### BACKGROUND OF THE INVENTION

Podophyllotoxin is a naturally occurring compound extracted from the mandrake plant. Recently two therapeutically useful semi-synthetic glycosides of podopyllotoxin, etoposide (also known as VP-16), shown below, and teniposide (also known as VM-26), have been developed.

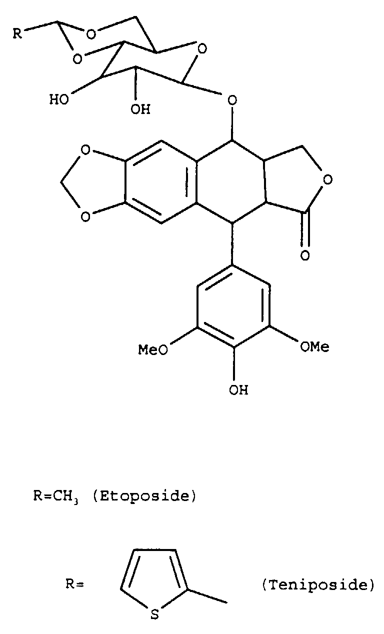

These compounds exhibit therapeutic activity in several human neoplasms, including small cell carcinomas of the lung, testicular tumors, Hodgkin's disease, Papillomavirus, and diffuse histiocytic lymphoma.

It is believed that these drugs block the catalytic activity of DNA topoisomerase II by stabilizing an enzyme-DNA complex in which the DNA is cleaved and covalently linked to the enzyme. See Chen, G.L., Yang, L., Rowe T.C., Halligan, B.D., Tewey, K., and Liu, L., J. Biol. Chem., 259, 13560 (1984); Ross, W., Rowe, T., Glisson, B., Yalowich, J., and Liu, L., Cancer Res., 44, 5857 (1984); Rowe, T., Kuppfer, G., and Ross, W., Biochem. Pharmacol., 34, 2483 (1985), which are all herein specifically incorporated by reference. By way of background, topoisomerases are enzymes which control the topological state of DNA. Type II topoisomerases catalyze DNA strand passage through transient double strand breaks in the DNA. The resulting change in the linking number of DNA allows these enzymes to mediate DNA interconversions, such as supercoiling and relaxation of supercoiling, catenation and decatenation, knotting, and unknotting. See Wang, J. C., Annu. Rev. Biochem., 54, 665 (1985) and Maxwell, A., and Gellert, M., Adv. Protein Chem., 38, 69 (1986), which are herein specifically incorporated by reference.

Type II DNA topoisomerase enzymes have been shown to be involved in a number of vital cellular processes, including DNA replication and transcription, and chromosomal segregation. These enzymes, therefore, are a critical target for the action of a wide variety of anticancer drugs, including etoposide and teniposide. The key step leading to cell death may be the capability of these drugs to block the catalytic activity of DNA topoisomerase II, as noted above.

Structure-activity studies have demonstrated a direct correlation between cytotoxicity, DNA breakage, and murine-derived topoisomerase II inhibition activities among the podophyllotoxin analogues. See Minocha, A., and Long, B., Biochem Res. Comm., 122, 165 (1984). The isolation and purification of human type II topoisomerase from lymphocytic leukemia cells has provided the means to use this enzyme as a target to investigate the structure-activity relationships among etoposide and related congeners.

It has been shown that the substitution of etoposide's glycosidic moiety by an 4-alkoxy group, as in 4'-demethyl-epipodophyllotoxin ethyl ether, preserves the inhibitory activity of DNA topoisomerase II intact at higher concentrations. See Thurston, L.S., Irie, H., Tani, S., Han, F. S., Liu, Z. C., Cheng, Y.C., and Lee, K. H., J. Med. Chem., 29, 1547 (1986), which is herein specifically incorporated by reference. However, it has also been shown that a series of 4-acyl congeners are less active, even though some of them possessed potent cytotoxicity. See Thurston, L. S., Imakura, Y., Haruna, M., Li, D. H., Liu, Z. C., Liu, S. Y., Cheng, Y. C., and Lee, K. H., J. Med. Chem., 31, (1988).

### Summary of the Invention

The present invention provides novel compounds which exhibit antitumor activity. The compounds are analogs of etoposide. More specifically, preferred compounds of the present invention are etoposide analogs wherein the glycosidic moiety is replaced by various substituents, such as a 4"-fluoroanilinyl chain. The compounds of the present invention have been shown to inhibit type II human topoisomerase and also to cause cellular protein-linked DNA breakage and, therefore, may be useful in the treatment of tumors. The compounds may also be useful in the treatment of papilloma virus.

In accordance with the present invention, there is provided a compound having the formula: 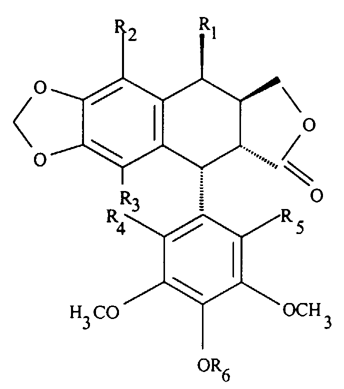 wherein
R₁ is 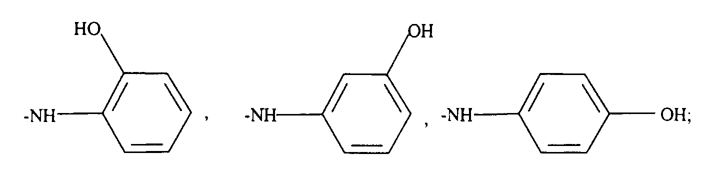
R₂ is H, or Br;
R₃ is H, or Br;
R₄ is H, or Br;
R₅ is H, or Br; and
R₆ is H, or -CH₃.

In a preferred embodiment, R₂ to R₆ are H.

The invention also provides a compound of the formula: 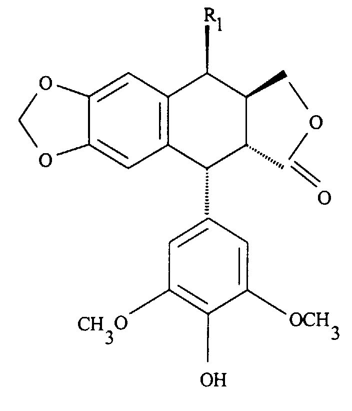 wherein R₁ is: 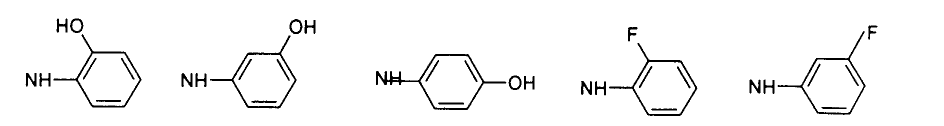 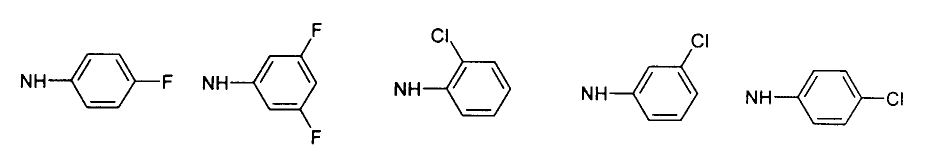 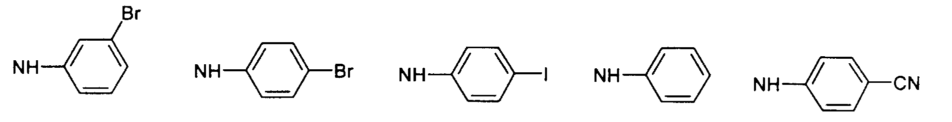 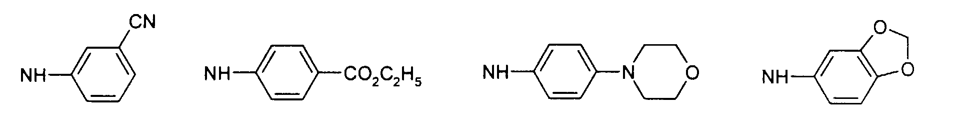 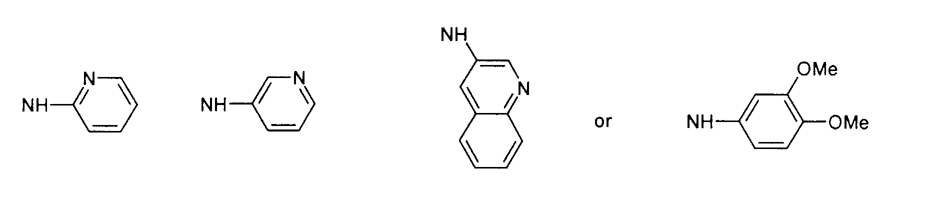 or compound of formula: 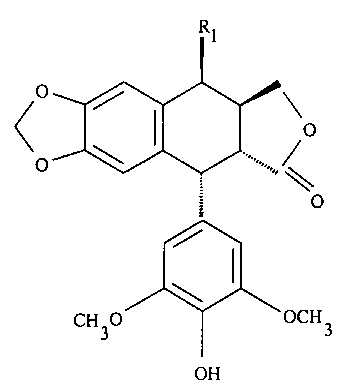 wherein R₁ is an arylamine of formula: 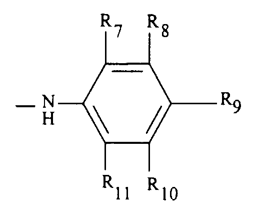 wherein
R₇ is H, OH, F, Cl, Br, CO₂CH₃, CO₂C₂H₅, CN, NO₂, NH₂, N(CH₃)₂, OCH₃, CH₂OH, CH₃, CF₃, CH₂CH₂OH, COCH₃, CH₂NH₂;
R₈ is H, OH, F, Cl, Br, CO₂CH₃, CO₂C₂H₅, CN, NO₂, NH₂, N(CH₃)₂, OCH₃, CH₂OH, CH₃, CF₃, CH₂CH₂OH, COCH₃, CH₂NH₂, CHOHCH₃, SCH₃;
R₉ is H, OH, F, Cl, Br, I, CHOCH₃, CO₂C₂H₅, CN, NO₂, NH₂, N(CH₃)₂, OCH₃, CH₂OH, CH₃, CF₃, CH₂CH₂OH, COCH₃, CH₂NH₂, N(CH₂CH₂OH)₂, 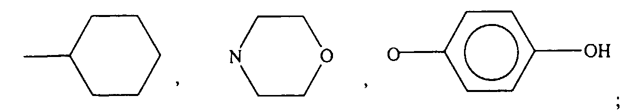
R₁₀ is H, F, Cl, OH, OCH₃, CO₂CH₃, CO₂C₂H₅, CH₃, CF₃, NO₂, NH₂, Cl;
R₁₁ is H, F, Cl, CH₃, CF₃, OH, OCH₃, NO₂;
R₉ and R₁₀ taken together are OCH₂O or OCH₂CH₂O.

In a further aspect of the invention, there is provided a pharmaceutical composition comprising a compound in accordance with the invention as described above.

Particularly preferred compounds include 4'-Demethyl-4β-[3"-hydroxyanilinyl]-4-desoxypodophyllotoxin; 4'-Demethyl-4β-[2"-hydroxyanilinyl]-4-desoxypodophyllotoxin; 4'-Demethyl-4β-[4"-hydroxyanilinyl)]-4-desoxypodophyllotoxin; 4'-Demethyl-4β-[2"-fluoroanilinyl]-4-desoxypodophyllotoxin; 4'-Demethyl-4β-[3"-fluoroanilinyl]-4-desoxypodophyllotoxin; 4'-Demethyl-4β-[4"-fluoroanilinyl]-4-desoxypodophyllotoxin; 4'-Demethyl-4β-[3",5"-difluoroanilinyl]-4-desoxypodophyllotoxin; 4'-Demethyl-4β-[4"-chloroanilinyl]-4-desoxypodophyllotoxin; 4'-Demethyl-4β-[4"-bromoanilinyl]-4-desoxypodophyllotoxin; 4'-Demethyl-4β-anilinyl-4-desoxypodophyllotoxin; 4'-Demethyl-4β-[4"-cyanoanilinyl]-4-desoxypodophyllotoxin; 4'-Demethyl-4β-[3"-cyanoanilinyl]-4-desoxypodophyllotoxin; 4'-Demethyl-4β-[4"-ethoxycarbonylanilinyl]-4-desoxypodophyllotoxin; 4'-Demethyl-4β-[4"-morpholinoanilinyl]-4-desoxypodophyllotoxin; 4'-Demethyl-4β-[3",4"-methylenedioxyanilinyl]-4-desoxypodophyllotoxin; 4'-Demethyl-4β-[3",4"-dimethoxyanilinyl]-4-desoxypodophyllotoxin; 4'-Demethyl-4β-[3"-pyridylamino]-4-desoxypodophyllotoxin; and 4'-Demethyl-4β-[3"-quinolinylamino]-4-desoxypodophyllotoxin.

Additional objects and advantages of the invention will be set forth in part in the description which follows, and in part will be obvious from the description, or may be learned from the practice of the invention.

### Description of The Preferred Embodiments

Reference will now be made in detail to the presently preferred embodiments of the invention, which together with the following examples, serve to explain the principles of the invention.

The compounds of the present invention are analogs of etoposide wherein the glycosidic moiety has been replaced. Compounds exhibiting potent inhibition of human DNA topoisomerase II result from replacing the glycosidic moiety with a substituted arylamine at the C-4β position. Inhibitory activity can also be increased by substituting the glycosidic moiety by chlorine, bromine, or an amino group at the C-4β position. It is believed that the stereochemistry of the 4β-substituents plays an important role in determining inhibitory potency. In general, β-isomers exhibit greater activity than the corresponding α-isomers. Another factor affecting the potency is the length of the substituent group at the C-4 position and the substitution on that group. This distance factor is different for halide and hydroxy substituted arylamines. For the hydroxy substituted arylamine, the meta position showed the most potency, while in the halogens, para substitution showed the most potency. A second potency factor regarding the halogen substituted aryl amines is the size of the halogen. Fluorine, the smallest has the most potency, while Iodine, the largest has the least. In addition, substitution of bromine at either one or more of the R₂, R₃, R₄, and R₅ positions will result in compounds having significant inhibitory activity. R₆ can be varied by substituting hydrogen with a methyl group. These modifications will produce changes in inhibitory activity which can be readily determined by assays known in the prior art through the exercise of routine skill in light of the teachings contained herein.

The compounds of the present invention were tested for their degree of inhibitory activity on human type II DNA topoisomerase, their effect on the formation of protein-linked DNA breakage, and their cytotoxicity. The inhibitory activity for compounds of the present invention correlated with the ability of the compounds to cause DNA strand breakage. However, the in vitro cytotoxicity of the compounds tested did not appear to correlate with the enzyme inhibitory activity and DNA strand break activity. The results of the tests on some of the compounds of the present invention are shown in Tables I and III. For a description of the assays used with respect to the compounds listed in Tables I and III see Thurston, L.S., Irie, H., Tani, S., Han, F. S., Liu, Z. C., Cheng, Y.C., and Lee, K. H., Antitumor Agents 78. Inhibition of Human DNA Topoisomerase II by Podophyllotoxin and α-Peltatin Analogues, J. Med. Chem. 29, 1547 (1986), and the references cited therein.

Table III illustrates the inhibitory activity, DNA strand breakage ability, as well as the cytotoxicity of etoposide and some of the compounds of the present invention. As shown in Table III the inhibitory activity of 4'-Demethyl-4β-[3"-hydroxyanilinyl]-4-desoxypodophyllotoxin (Example 33), 4'-Demethyl-4β-[2"-hydroxyanilinyl]-4-desoxypodophyllotoxin (Example 34), 4'-Demethyl-4β-[4"-hydroxyanilinyl]-4-desoxypodophyllotoxin (Example 35), 4'-Demethyl-4β-[3"-fluoroanilinyl]-4-desoxypodophyllotoxin (Example 26); 4'-Demethyl-4β-[4"-fluoroanilinyl]-4-desoxypodophyllotoxin (Example 28); 4'-Demethyl-4β-[4"-chloroanilinyl]-4-desoxypodophyllotoxin (Example 38); 4'-Demethyl-4β-anilinyl-4-desoxypodophyllotoxin (Example 19); 4'-Demethyl-4β-[4"-cyanoanilinyl]-4-desoxypodophyllotoxin (Example 20); 4'-Demethyl-4β-[3"-cyanoanilinyl]-4-desoxypodophyllotoxin (Example 21); 4'-Demethyl-4β-[4"-ethoxycarbonylanilinyl]-4-desoxypodophyllotoxin (Example 22); 4'-Demethyl-4β-[4"-morpholinoanilinyl]-4-desoxypodophyllotoxin (Example 23); 4'-Demethyl-4β-[3",4"-methylenedioxyanilinyl]-4-desoxypodophyllotoxin (Example 24); 4'-Demethyl-4β-[3",4"-dimethoxyanilinyl]-4-desoxypodophyllotoxin (Example 25); 4'-Demethyl-4β-[3"-pyridylamino]-4-desoxypodophyllotoxin (Example 30); 4'-Demethyl-4β-[2"-pyridylamino]-4-desoxypodophyllotoxin (Example 31); and 4'-Demethyl-4β-[3"-quinolinylamino]-4-desoxypodophyllotoxin (Example 32) equals or exceeds that of etoposide. In addition, as shown in Table III, the DNA strand breakage abilities of 4'-Demethyl-4β-[3-hydroxyanilinyl]-4-dexoypodophyllotoxin (Example 33), 4'-Demethyl-4β-[2-hydroxyanilinyl]-4-desoxypodophyllotoxin (Example 34), 4'-Demethyl-4β-[4-hydroxyanilinyl]-4-desoxypodophyllotoxin (Example 35), 4'-Demethyl-4β-[3"-fluoroanilinyl]-4-desoxypodophyllotoxin (Example 26); 4'-Demethyl-4β-[2"-fluoroanilinyl]-4-desoxypodophyllotoxin (Example 27); 4'-Demethyl-4β-[4"-fluoroanilinyl]-4-desoxypodophyllotoxin (Example 28); 4'-Demethyl-4β-[3",5"-difluoroanilinyl]-4-desoxypodophyllotoxin (Example 29); 4'-Demethyl-4β-anilinyl-4-desoxypodophyllotoxin (Example 19); 4'-Demethyl-4β-[4"-cyanoanilinyl]-4-desoxypodophyllotoxin (Example 20); 4'-Demethyl-4β-[3"-cyanoanilinyl]-4-desoxypodophyllotoxin (Example 21); 4'-Demethyl-4β-[4"-ethoxycarbonylanilinyl]-4-desoxypodophyllotoxin (Example 22); 4'-Demethyl-4β-[4"-morpholinoanilinyl]-4-desoxypodophyllotoxin (Example 23); 4'-Demethyl-4β-[3",4"-methylenedioxyanilinyl]-4-desoxypodophyllotoxin (Example 24); 4'-Demethyl-4β-[3",4"-dimethoxyanilinyl]-4-desoxypodophyllotoxin (Example 25); 4'-Demethyl-4β-[3"-pyridylamino]-4-desoxypodophyllotoxin (Example 30); 4'-Demethyl-4β-[3"-quinolinylamino]-4-desoxypodophyllotoxin (Example 32); and 4'-Demethyl-4β-[4"-bromoanilinyl]-4-desoxypodophyllotoxin (Example 40) greatly exceeds that of etoposide.

Preparation of compounds within the scope of the present invention appear in the following examples.

### EXAMPLE 1 (Reference Example)

### Preparation of 4'-Demethylepipodophyllotoxin.

5 g. (12.1 mmol) of podophyllotoxin were dissolved in 75 ml of anhydrous CH₂Cl₂. Dry hydrogen bromide gas was then bubbled through the solution to saturation. The reaction mixture was then capped and allowed to stand at room temperature for 48 hours. Removal of the solvent yielded a residue which was then treated with 25 ml of water, 50 ml of acetone and 5 g. of BaCO₃, and refluxed for one hour. The reaction mixture was extracted with chloroform and chromatographed on a silica gel column. The product was obtained by elution with chloroform-methanol (30:1) and recrystallized from CH₂Cl₂/ethylether to give 2.5 g. (52%) of 4'-Demethylepipodophyllotoxin. Spectral data agreed with that described by Kuhn, M., Keller-Julsen, C., and von Wartburg, A., Helv. Chim. Acta, 52, 944 (1969).
(See Scheme 1)

### EXAMPLE 2 (Reference example)

### Preparation of 4'-Demethylpodophyllotoxin.

4'-Demethylpodophyllotoxin was obtained using the silica gel column of Example 1 by further elution with chloroform-methanol (30:1). The product obtained by elution was crystallized from acetone in 5% (0.5 g) yield. Spectral data agreed with that described by Kuhn, M., and von Wartburg, A., Helv. Chim. Acta, 52, 948 (1969) (hereinafter Kuhn and von Wartburg).

### EXAMPLE 19 - 41

### Preparation of 4'demethyl-4β-(arylamino)-4-desoxypodophyllotoxins (19-41). (Scheme VIII)

The 4'-demethyl-4β-(arylamino)-4-deoxypodopohyllotoxins specified in examples 19 - 41 were prepared according to the following procedure:

A solution of 4'-demethylepipodophyllotoxin (10 g, 24 mmol) in 250 ml of dry dichloromethane was kept at 0°C, and dry hydrogen bromide gas was bubbled through the solution. After 30 min., nitrogen was bubbled through the solution to drive off excess hydrogen bromide. The solution was then evaporated in vacuum to dryness by means of azeotropic distillation with benzene.

The desired product (11.5 g) was obtained and then used for the next step reaction without any further purification.

Spectral data agreed with that described by M. Kuhn and A. Von Wartburg. Helv. Chim. Acta, 52, 944 (1969).

A solution containing 4'-demethyl-4β-bromo-4-deoxypodophyllotoxin (300 mg, 0.65 mmol), barium carbonate (153 mg, 0.78 mmol) and the appropriate arylamines (0.78 mmol) in 7 ml of dry 1, 2-dichloroethane was stirred overnight at room temperature. The reaction mixture was filtered, diluted with ethyl acetate, washed with water, dried and purified via column chromatography. The products (19-41) obtained in the examples had the characteristics listed below.

### EXAMPLE 19

### 4'-Demethyl-4β-anilinyl-4-desoxypodophyllotoxin.

Crystals from methanol; mp 172-173°, [α]²⁵_{D}-120° (C=1.0, CHCI₃); IR (KBr) 3500 (OH), 3360 (NH), 2900 (aliphatic C-H), 1755 (lactone), 1595, 1500 and 1475 (aromatic c=c) cm⁻¹: ¹H NMR CDCl₃, δ 7.22 (t, J=7.5 Hz, 2H, 3", 5"-H), 6.80 (m, 2H, 4" -H and 5-H), 6.50 (m, 3H, 2"-H, 6"-H and 8-H), 6.33 (s, 2H, 2', 6'-H), 5.97 (AB_{q}, J=1.3, 3.6 Hz, OCH₂O), 5.42 (s, 1H, exchangeable, 4'-OH), 4.68 (br, 1H, 4-H), 4.60 (d, J=4.9 Hz, 1-H), 4.38 (t, J=8.4 Hz, 1H, 11-H), 4.01 (t, J=8.4 Hz, 1H, 11-H), 3.85 (br, 1H, exchangeable, NH) , 3.79 (s, 6H, 3', 5'-OCH₃), 3.16 (dd, J=5.0, 14.0 Hz, 1H, 2-H), 3.00 (m, 1H, 3-H).
Anal. (C₂₇H₂₅NO₇), C.H.N.

### EXAMPLE 20

### 4'-Demethyl-4β-[4"-cyanoanilinyl]-4-desoxypodophyllotoxin.

Crystals from ethanol; mp 187-189°, [α]²⁵_{D}-145° (C=1.0, CHCI₃); IR (KBr) 3500 (OH), 3360 (NH), 2890 (aliphatic C-H), 2210 (lactone), 1600, 1510 and 1475 (aromatic C=C) cm⁻¹: ¹H NMR CDCI, δ 7.50 (d, J=8.7 Hz, 2H, 3", 5"-H), 6.74 (s, 1H, 5-H), 6.57 (d, J=8.7 Hz, 2H, 2", 6"-H), 6.55 (s, 1H, 8-H), 6.32 (s, 2H, 2', 6'-H), 5.99 (AB_{q}, J-1.2, 8.3 Hz, 2H, OCH₂O), 5.44 (s, 1H, exchangeable, 4'-OH), 4.78 (m, 1H, exchangeable, NH), 4.63 (d, J=4.2 Hz, 1H, 4-H), 4.36 (m, 2H, 11-H), 3.85 (m, 1H, 1-H), 3.79 (s, 6H, 3', 5'-OCH₃), 3.09 (dd, 1H, 2-H), 3.05 (m, 1H, 3-H).
Anal. (C₂₈H₂₄NO₇), C.H.N.

### EXAMPLE 21

### 4'-Demethyl-4β-[3"-cyanoanilinyl]-4-desoxypodophyllotoxin.

Crystals from methanol; mp 191-192°, [α]²⁵_{D}-117° (C=0.33, CHCI₃); IR (KBr) 3450 (OH), 3360 (NH), 2900 (aliphatic C-H), 2225 (CN), 1750 (lactone), 1595, 1500 and 1450 (aromatic C=C) cm⁻¹; ¹H NMR CDCI₃ δ 7.31 (t, J=7.6 Hz, 5"-H), 7.07 (d, J-7.6 Hz, 4"-H), 6.80 (d, 2H, 2"-H and 6"-H), 6.74 (s, 1H, 5-H), 6.55 (s, 1H, 8-H), 6.33 (s, 2H, 2', 6'-H), 6.00 (d, J=7.0 Hz, 2H, OCH₂O), 5.48 (s, 1H exchangeable, 4'-OH), 4.69 (d, J=3.8 Hz, 1H, 4-H), 4.62 (d, J-4.5 Hz, 1H, 2-H), 4.41 (t, J=8.5 Hz, 1H, 11-H), 3.92 (t, J=8.5 Hz, 1H, 11-H), 3.81 (s, 6H, 3', 5'-OCH₃), 3.14-3.00 (m, 2H, 2-H and 3-H).
Anal. (C₂₈H₂₄N₂O₇)·1/2 H₂O, C.H.N.

### EXAMPLE 22

### 4'-Demethyl-4β-[4"-ethoxycarbonylanilinyl]-4-desoxypodophyllotoxin.

Crystals from ethanol; mp 270-271°, [α]²⁵_{D}-145° (C=0.33, CHCI₃); IR (KBr) 3500 (OH), 3370 (NH), 2940 (aliphatic C-H), 1762 (lactone), 1695 (ester), 1610, 1520 and 1480 (aromatic C=C) cm⁻¹: ¹H NMR CDCI₃ δ 7.92 (d, J=8.8 Hz, 2H, 3", 5"-H), 6.77 (s, 1H, 5-H), 6.55 (d, J=8.8 Hz, 2H, 2", 6"-H), 6.54 (s, 1H, 8-H), 6.33 (s, 2H, 2', 6'-H), 5.99 (AB_{q}, J=1.1, 8.2 Hz, 2H, OCH₂O), 5.44 (s, 1H, exchangeable, 4'-OH), 4.78 (d, J-3.3 Hz, 1H, 4-H), 4.62 (d, J=4.5 Hz, 1H, 1-H), 4.40 (m, 2H, 4-H and 11-H), 4.37 (q, J=7.1 Hz, 2H, CO₂CH₂CH₃), 4.32 (d, J=7.1, 1H, exchangeable, NH), 3.92 (t, J=7.5 Hz, 1H, 11-H), 3.80 (s, 6H, 3', 5'-OCH₃), 3.10 (dd, 1H, 2-H), 3.08 (m, 1H, 3-H), 1.38 (t, J=7.1 Hz, 3H, CO₂CH₂CH₃).
Anal. (C₃₀H₂₉NO₉), C.H.N.

### EXAMPLE 23

### 4'-Demethyl-4β-[4"-morpholinoanilinyl]-4-desoxypodophyllotoxin.

Crystals from ethanol; mp 235-237°, [α]²⁵_{D}-129° (C=1, CHCI₃); IR (KBr) 3500 (OH), 3300 (NH), 2880 (aliphatic C-H), 1755 (lactone), 1620, 1510 and 1475 (aromatic C=C) cm⁻¹: ¹H NMR CDCI₃ δ 6.86 (d, J=9.5 Hz, 2H, 3", 5"-H), 6.76 (s, 1H, 5-H), 6.52 (br, 3H, 8-H and 2", 6"-H), 6.35 (s, 2H, 2', 6'-H), 5.96 (d, J=66.7 Hz, 2H, OCH₂O), 5.44 (s, 1H, exchangeable, 4'-OH), 4.61 (m, 2H, 4-H and 1-H), 4.37 (t, J=7.0 Hz, 1H, 11-H), 4.08 (t, J-7.0 Hz, 1H, 11-H), 3.82 (br, 4H, ), 3.80 (s, 6H, 3', 5'-OCH₃), 3.22-2.90 (m, 2H, 2-H, 3-H and 4H, 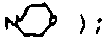 Anal. (C₃₁H₃₂N₂O₈), C.H.N.

### EXAMPLE 24

### 4'-Demethyl-4β-[3", 4"-(methylenedioxy)anilinyl]-4-desoxypodophyllotoxin.

Crystals from methanol; mp 247-2497°, [α]²⁵_{D}-126° (C=1, CHCI₃); IR (KBr) 3500 (OH), 3340 (NH), 2900 (aliphatic C-H), 1752 (lactone), 1605, 1496 and 1475 (aromatic C=C) cm⁻¹: ¹H NMR CDCI₃, δ 6.76 (s, 1H, 5-H), 6.68 (d, J=8.1 Hz, 1H, 5"-H), 6.52 (s, 1H, 8-H), 6.33 (s, 2H, 2', 6'-H), 6.17 (d, J=1.2 Hz, 1H, 2"-H), 5.96 (q, J=1.2, 8.1 Hz, 3H, 6"H and OCH₂O), 5.90 (s, 2H, 7"-H), 5.43 (s, 1H exchangeable, 4'-OH), 4.59 (d, J=4.9 Hz, 1H, 4-H), 4.56 (d, J=3.9 Hz, 1H, 1-H), 4.37 (t, 1H, 11-H), 4.05 (t, 1H, 11-H), 3.79 (s, 6H, 3', 5'-OCH₃), 3.15 (dd, 1H, 2-H), 2.95 (m, 1H, 3-H).
Anal. (C₂₈H₂₅NO₉), C.H.N.

### EXAMPLE 25

### 4'-Demethyl-4β-[3", 4"-dimethoxyanilinyl]-4-desoxypodophyllotoxin.

Crystals from methanol; mp 233-234° (dec); [α]²⁵_{D}-118° (C=1, CHCI₃); IR (KBr) 3500 (OH), 3360 (NH), 2920 (aliphatic C-H), 1770 (lactone), 1605, and 1505 (aromatic C=C) cm⁻¹: ¹H NMR CDCI₃, δ 6.78 (s, 1H, 5-H), 6.75 (d, J=8.5 Hz, 1H, 5"-H), 6.53 (s, 1H, 8-H), 6.34 (s, 2H, 2', 6'-H), 6.17 (s, 1H, 2"-H), 6.05 (d, J=8.5 Hz, 1H, 6"-H), 5.96 (d, J=2.4 Hz, 2H, OCH₂O), 5.43 (s, exchangeable, 4'-OH), 4.60 (d, 2H, 4-H and 1-H), 4.38 (t, J=8.3 Hz, 1H, 11-H), 4.05 (t, J=8.3 Hz, 1, 11-H), 3.83 (s, 3H, 4"-OCH₃), 3.81 (s, 3H, 3"-OCH₃), 3.80 (s, 6H, 3', 5'-OCH₃), 3.18 (dd, J=5.0, 14.0 Hz, 1H, 2-H), 2.96 (m, 1H, 3-H).
Anal. (C₂₈H₂₅NO₉), C.H.N.

### EXAMPLE 26

### 4'-Demethyl-4β-[3"-fluoroanilinyl]-4-desoxypodophyllotoxin.

Crystals from methanol; mp 201-203°C (dec.);[α]²⁵D-132° (c = 1, CHCl₃); IR (KBr) 3500 (OH), 3360 (NH), 2900 (aliphatic C-H), 1750 (lactone), 1605, 1500 1475 (aromatic C=C) cm⁻¹; ¹HNMR (CDCl₃) δ 7.15 (t, J = 7.4 Hz, 1H, 5"-H), 6.76 (s, 1H, 5-H), 6.53 (s, 1H, 8-H), 6.49 (dd, H = 1.2, 7.4 Hz, 1H, 4"-H), 6.40 (s, 2H, 2', 6'-H), 6.32 (d, J = 1.2 Hz, 1H, 2"-H), 6.24 (dd, J = 1.2, 7.4 Hz, 1H, 6"-H), 5.97 (ABq, J = 1.2, 7.9 Hz, 2H OCH₂O), 5.44 (s, 1H, exchangeable, 4'-OH), 4.67 (s, 1H, exchangeable, NH), 4.63 (d, J = 4.0 Hz, 1H, 4-H), 4.59 (d, J = 5.0 Hz, 1H, 1-H), 4.39 (t, J = 8.5 Hz, 1H, 11-H) 3.98 (t, J = 8.5 Hz, 1H, 11-H), 3.79 (s, 6H, 3',5'-OCH₃), 3.11 (dd, J = 5.0, 14.0 Hz, 1H, 2-H), 3.00 (m, 1H, 3-H). Anal. calcd for C₂₇H₂₄FNO₇:C,65.71;H, 4.90; N, 2.84. Found: C, 66.81; H, 4.94; N, 2.79.

### EXAMPLE 27

### 4'-Demethyl-4β-[2"-fluoroanilinyl]-4-desoxypodophyllotoxin.

Crystals from methanol; mp 197-198°C;[α]²⁵D-128° (c = 0.25, CHCl₃); IR (KBr) 3500 (OH), 4480 (NH), 2890 (aliphatic C-H), 1755 (lactone), 1610, 1505 and (aromatic C=C) cm⁻¹; ¹H NMR (CDCl₃) δ 7.04 (m, 2H, 3",6"-H), 6.76 (s, 1H, 5H), 6.72 (m, 1H 5"-H), 6.60 (t, J = 7.2 Hz, 1H, 4"-H), 6.54 (s, 1H, 8-H), 6.34 (s, 2H, 2',6'-H), 5.97 (d, J = 7.3 Hz, 2H, OCH₂O), 5.46 (s, 1H, exchangeable, 4'-OH), 4.69 (d, J = 4.2 Hz, 1H, 4-H), 4.62 (d, J = 4.9 Hz, 1H, 1-H), 4.38 (t, J = 8.2 Hz, 1H, 11-H) 4.10 (t, 1H, exchangeable, NH), 3.82 (t, J = 8.2 Hz, 1H, 11-H) 3.79 (s, 6H, 3',5'-OCH₃), 3.15 (dd, J = 5.0, 14.0 Hz, 1H, 2-H), 3.00 (m, 1H, 3-H). Anal. calcd for C₂₇H₂₄FNO₇:C,65.71;N, 4.90; N, 2.84. Found: C, 66.80; H, 4.95; N, 2.84.

### EXAMPLE 28

### 4'-Demethyl-4β-[4"-fluoroanilinyl]-4-desoxypodophyllotoxin.

Crystals from ethanol; mp 176-177°C;[α]²⁵D-100° (c = 0.8, CHCl₃); IR (KBr) 3540 (OH), 3420 (NH), 2900 (aliphatic C-H), 1740 (lactone), 1610, 1500 1480 (aromatic C=C) cm⁻¹; ¹HNMR (CDCl₃) δ 6.94 (t, J = 6.7, 2-H, 3",5",-H), 6.75 (s, 1H, 5-H), 6.53 (s, 1H, 8H), 6.49 (q, J = 2.2, 6.2 Hz, 2H, 2",6"-H), 6.33 (s, 2H, 2',6'-H), 5.96 (ABq, J = 1.2, 7.5 Hz, 2H OCH₂O), 5.43 (s, 1H, exchangeable, 4'-OH), 4.60 (d, 2H, 4-H and 1-H), 4.37 (t, J = 7.5 Hz, 1H, 11-H) 3.99 (t, J = 7.5 Hz, 1H, 11-H), 3.79 (s, 6H, 3',5'-OCH₃), 3.73 (br, 1H, exchangeable, NH), 3.13 (t, J = 5.0, 14.0 Hz, 1H, 2-H), 3.00 (m, 1H, 3-H). Anal. C₂₇H₂₄FNO₇), C.H.N.

### EXAMPLE 29

### 4'-Demethyl-4β-[3", 5"-difluoroanilinyl]-4-desoxypodophyllotoxin.

Crystals from ethanol; mp 180-183°C;[α]²⁵D-132° (c = 0.33, CHCl₃); IR (KBr) 3500 (OH), 3370 (NH), 2890 (aliphatic C-H), 1750 (lactone), 1620, 1590, 1500 and 1475 (aromatic C=C) cm⁻¹; ¹HNMR (CDCl₃) δ 6.75 (s, 1H, 5-H), 6.54 (s, 1H, 8-H), 6.32 (s, 2H, 2',6'-H), 6.23 (m, 1H, 4"-H), 6.07 (m, 2H, 2",6"-H), 5.98 (ABq, J = 1.3, 9.0 Hz, 2H OCH₂O), 5.45 (s, 1H, exchangeable, 4'-OH), 4.61 (m, 2H, 4-H and 1-H), 4.39 (t, J = 8.5 Hz, 1H, 11-H) 4.10 (d, J = 6.1 Hz, 1H, 1H, exchangeable, NH), 3.85 (t, J = 8.5 Hz, 1H, 11-H), 3.81 (s, 6H, 3',5'-OCH₃), 3.08 (dd, J = 4.8, 14.1 Hz, 1H, 2-H), 3.02 (m, 1H, 3-H). Anal. C₂₇H₂₃NF₂O₉). C.H.N.

### EXAMPLE 30

### 4'-Demethyl-4β-[3"-pyridylamino]-4-desoxypodophyllotoxin.

Crystals from ethanol; mp 179-181° (dec); [α]²⁵_{D}-99° (C=0.33, CHCI₃); IR (KBr) 3500 (OH), 3350 (NH), 2900 (aliphatic C-H), 1765 (lactone), 1575, 1500 and 1470 (aromatic C=C=N) cm⁻¹: ¹H NMR CDCI₃ δ 8.08 (d, J=5.5 Hz, 1H, 6"-H), 8.02 (br, 1H, 2"-H), 7.16 (m, 1H, 5"-H), 6.85 (dd, 1H, 4"-H), 6.75 (s, 1H, 5-H), 6.55 (s, 1H, 8-H), 6.32 (s, 2H, 2', 6'H), 5.98 (AB_{q}, J=1.3, 7.3 Hz, 2H, OCH₂O), 4.65 (d, J=4.9 Hz, 1H, 4-H), 4.60 (m, 1H, 1-H), 4.20 (t, J=8.2 Hz, 1H, 11-H), 3.90 (m, 2H, 11-H and NH), 3.80 (s, 6H, 3', 5'-OCH₃), 3.18 (dd, J=5.0, 14.1 Hz, 1H, 2-H), 3.03 (m, 1H, 3-H).
Anal. (C₂₆H₂₄N₂O₇) 1/2 H₂O, C.H.N.

### EXAMPLE 31

### 4'-Demethyl-4β-[2"-pyridylamino]-4-desoxypodophyllotoxin.

Crystals from ethanol; mp 215-218° (dec); [α]²⁵_{D}-82° (C=0.33, CHCI₃); IR (KBr) 3500 (OH), 3360 (NH), 2950 (aliphatic C-H), 1760 (lactone), 1690, 1645, 1600 and 1460 (aromatic C=C=N) cm⁻¹: ¹H NMR CDCI₃ δ 8.11 (d, 1H, 4"-H), 7.45 (m, 1H, 4"-H), 6.81 (s, 1H, 5-H), 6.67 (m, 1H, 5"-H), 6.55 (s, 1H, 8-H), 6.45 (d, 1H, 3"-H), 6.34 (s, 2H, 2', 6'-H), 5.97 (AB_{q}, J=1.3, 6.7 Hz, 2H, OCH₂O), 5.43 (br, 1H, exchangeable, 4'-OH), 5.35 (m, 1H, exchangeable, NH), 4.60 (d, J=4.2 Hz, 1H, 4-H), 4.24 (m, 2H, 1-H and 11NH), 3.85 (m, 1H, 11H), 3.78 (s, 6H, 3', 5'-OCH₃), 3.05 (m, 2H, 2-H and 3-H).
Anal. (C₂₆H₂₄N₂O₇), 1/2 H₂O, C.H.N.

### EXAMPLE 32

### 4'-Demethyl-4β-[3"-quinolinylamino]-4-desoxypodophyllotoxin.

Crystals from ethanol-ether; mp 243-246° (dec); [α]²⁵_{D}-179° (C=0.5, CHCI₃); IR (KBr) 3460 (OH), 3380 (NH), 2900 (aliphatic C-H), 1775 (lactone), 1605, 1510 and 1480 (aromatic C=C=N) cm⁻¹: ¹H NMR CDCI₃ δ 8.46 (d, J=2.9 Hz, 2"-H), 7.97 (m, 1H, 4"-H), 7.65 (m, 1H, 7"-H), 7.48 (m, 2H, 5", 6"-H), 6.99 (d, J=2.9 Hz, 8'-H), 6.76 (s, 1H, 5-H), 6.57 (s, 1H, 8-H), 6.35 (s, 2H, 2', 6'-H), 5.99 (AB_{q}, J=1.1, 8.0 Hz, 2H, OCH₂O), 5.48 (s, 1H, exchangeable, 4'-OH), 4.78 (d, J=4.8 Hz, 1H, 1-H), 4.45 (t, 1H, 11-H), 4.23 (d, 1H, exchangeable, NH), 3.99 (t, 1H, 11-H), 3.81 (s, 6H, 3', 5'-OCH₃), 3.15 (m, 2H, 2-H and 3-H).
Anal. (C₃₀H₂₆N₂O₇) 1/2H₂O, C.H.N.

### EXAMPLE 33

### 4'-Demethyl-4β-[3"-hydroxyanilinyl]-4-desoxypodophyllotoxin.

Amorphous powder from ether: mp 163-166°C; IR (KBr) 3480 (OH), 3380 (NH), 2900 (aliphatic CH), 1750 (lactone), 1590, 1475 (aromatic C=C) cm⁻¹ ; ¹H NMR (CDCl₃) δ 7.05(t,J=8Hz,1H,5"-H), 6.78 (s,1H,5-H), 6.52(s,1H,8-H), 6.33(s,2H,2',6'-H), 6.24 (dd,J=2.2, 8Hz,1H,4"-H), 6.15(dd,J=1.7, 8Hz,1H,6"-H), 6.07(t,J=2.2Hz,1H,2"-H), 5.97(d,J=4.4Hz,2H, OCH₂O), 5.43(s, exchangeable), 4.82(s, exchangeable), 4.65 (d,J=3.9Hz,1H,4-H), 4.58(d,J=4.8Hz,1H,1-H), 4.37(t,J=8.7Hz,1H,11-H), 4.0(t,J=8.7Hz,1H,11-H), 3.79(s,6H,3'5'-OCH₃), 3.1(dd, J=4.8, 14.1 Hz, 1H, 2-H), 2.98 (m,1H,3-H); MS, m/z=491 (m+). Anal. Calcd for C₂₇H₂₅NO₈.H₂O: C,63.65;H,5.30. Found: C,63.35; H,5.44.

### EXAMPLE 34

### 4'-Demethyl-4β-[2"-hydroxyanilinyl]-4-desoxypodophyllotoxin.

Amorphous crystals from ether: mp 175°C; IR (KBr) 3360 (OH, NH), 2900 (aliphatic C-H), 1750 (lactone, 1600, 1475 (aromatic C=C) cm-¹; ¹H NMR (CDCI₃) δ 6.88(t,J=7.4H₂,1H,4"-H), 6.78(s,1H,5-H), 6.65(m,2H,3",6"-H), 6.5(m,2H,8-H,5"-H), 6.35(s,2H,2'6'-H), 5.96(AB_{q} J=1.2Hz,3.5Hz,2H,OCH₂O), 5.44(s.exchangeable), 5.10(s, exchangeable), 4.67(d,J=4Hz,1H,4-H), 4.61(d,J=4.8Hz,1H,1-H), 4.38(t,J=8.5Hz,1H,11-H), 3.98(t,J=8.5Hz,1H,11-H), 3.79(s,6H,3',5'-OCH₃), 3.24 (dd,J=4.8,14Hz,1H,2-H), 3.02(m,1H,3-H), MS, m/z=491(m+). Anal. Calcd for C₂₇H₂₅NO₈: C,65.99; H,5.09. Found: C, 65.85; H,5.18.

### EXAMPLE 35

### 4'-Demethyl-4β-[4"-hydroxyanilinyl]-4-desoxypodophyllotoxin.

Amorphous powder from ether mp 162-165°C;IR (KBr) 3525 (OH), 3345 (NH), 3010 (aromatic CH), 2900 (aliphatic CH), 1745 (lactone), 1600, 1475 (aromatic C=C) cm⁻¹;¹H NMR (DMSO d₆, D₂O exchange) δ 6.69 (s,1H,5-H), 6.55(s,4H,2",3",5",6"-H), 6.48(s,1H,8-H), 6.23(s,2H,2'6'-H), 5.94(d,J=9.7Hz,2N,O-CH2-O), 4.68(d,J=4.3Hz,1H,4-H), 4.46(d,J=5.4Hz,1H,1-H), 4.29(t,J=7.6,1H,11-H), 3.76(t,J=7.6Hz,1H,11-H), 3.61(s,6H,3',5'-OCH₃), 3.28(dd,J=5.4,15.8Hz,1H,2-H), 2.95(m,1H,3-H).

### EXAMPLE 36

### 4'-Demethyl-4β-[2"-chloroanilinyl]-4-desoxypodophyllotoxin.

Crystals from ethyl acetate/ether, mp 253-255°C;[α]²⁵D-90° (c = 1.0, CHCl₃); IR (KBr) 3500 (OH), 3450 (NH), 2895 (aliphatic CH), 1751 (lactone), 1590, 1500 1472 (aromatic C=C) cm⁻¹; ¹H NMR (CDCl₃) 7.31 (dd, J = 1.4, 7.9 Hz, 1H, 3"-H), 7.18 (t, J = 8.8, Hz, 1H, 5"-H), 6.76 (s, 1H, 5-H), 6.73 (t, J = 9.0 Hz, 4"-H) 6.58 (d, J = 8.2 Hz, 1H, 6"-H), 6.54 (s, 1H, 8-H), 6.35 (s, 2H, 2',6'-H), 5.98 (ABq, J = 1.2, 4.2 Hz, 2H OCOCH₂O), 5.44 (s, 1H, exchangeable, 4'-OH), 4.73 (t, J = 4.9 Hz, 1H, 4-H) 4.64 (d, J = 4.9 Hz, 1H, 1-H), 4.49 (d, J = 6.0 Hz, 1H, exchangeable, NH), 4.36 (t, J = 8.3 Hz, 1H, 11-H), 3.91 (t, J = 8.3, Hz, 1H, 11-H), 3.80 (s, 6H, 3,'5'-OCH₃), 3.17 (dd, J = 4.8, 14.0 Hz, 1H, 2-H), 3.04 (m, 1H, 3-H). Anal. C₂₇H₂₄CINO₇ C.H.N.

### EXAMPLE 37

### 4'-Demethyl-4β-[3"-chloroanilinyl]-4-desoxypodophyllotoxin.

Crystals from ethyl acetate/ether, mp 174-176°C;[α]²⁵D-112° (C = 1.0, CHCl₃); IR (KBr) 3500 (OH), 3360 (NH), 2920 (aliphatic CH), 1752 (lactone), 1580, and 1452 (aromatic C=C) cm⁻¹; ¹H NMR (CDCl₃) 7.12 (t, J = 8.1, Hz, 1H, 5"-H), 6.76 (s, 1H, 5-H), 6.74 (dd, J = 1.0, 8.1 Hz, 1H 4"-H), 6.53 (br, 2H, 8-H and 2"-H), 6.42 (dd, J = 1.6, 6.5 Hz, 1H, 6"-H), 6.33 (s, 2H, 2',6'-H), 5.97 (ABq, J = 1.0, 8.7 Hz, 2H OCH₂O), 5.43 (s, 1H, exchangeable, 4'-OH), 4.66 (br, 1H, 4-H), 4.59 (d, J = 4.8 Hz, 1H, 1-H), 4.39 (t, J = 7.7 Hz, 1H, 11-H), 3.99 (t, J = 7.7 Hz, 1H, 11-H) 3.96 (br, 1H, exchangeable, NH), 3.79 (s, 6H, 3,'5'-OCH₃), 3.11 (dd, J = 5.8, 14.0 Hz, H, 2-H), 3.01 (m, 1H, 3-H). Anal. C₂₇H₂₄CINO₇) C.H.N.

### EXAMPLE 38

### 4'-Demethyl-4β-[4"-chloroanilinyl]-4-desoxypodophyllotoxin.

Crystals from ethyl acetate/ether, mp 253-255°C;[α]²⁵D-125° (c = 0.75 CHCl₃); IR (KBr) 3500 (OH), 3360 (NH), 2920 (aliphatic CH), 1758 (lactone), 1605, 1590 and 1475 (aromatic C=C) cm⁻¹; ¹H NMR (CDCl₃) δ 7.17 (d, J = 8.7, Hz, 2H, 3",5"-H), 6.74 (s, 1H, 5-H), 6.53 (s, 1H, 8-H), 6.48 (d, J = 8.7 Hz, 2H, 2",6"-H), 6.32 (s, 2'6'-H), 5.96 (ABq, J = 1.0, 6.8 Hz, 2H OCH₂O), 5.43 (s, 1H, exchangeable, 4'-OH), 4.63 (d, J = 4.2 Hz, 1H, 4-H), 4.59 (d, J = 4.9 Hz, 1H, 1-N), 4.38 (t, J = 8.0 Hz, 1H, 11-H) 3.96 (t,J = 8.0 Hz, 1H, 11-H), 3.79 (s, 6H 3,'5'-OCH₃), 3.12 (dd, J = 4.9, 14.1 Hz, H, 2-H), 2.99 (m, 1H, 3-H). Anal. C₂₇H₂₄CINO₇) C.H.N.

### EXAMPLE 39

### 4'-Demethyl-4β-[3"-bromoanilinyl]-4-desoxypodophyllotoxin.

Crystals from methanol/ether; mp 177-179°C;[α]²⁵D-105° (c = 1, CHCl₃); IR (KBr) 3450 (OH), 3340 (NH), 2900 (aliphatic CH), 1740 (lactone), 1590, 1500 and 1475 (aromatic C=C) cm¹; ¹H NMR (CDCl₃) δ 7.07 (t, J = 8.0, Hz, 1H, 5"-H), 6.90 (dd, J = 0.9, 7.9 Hz, 1H, 4"-H), 6.75 (s, 1H, 5-H), 6.70 (br, 1H, 2"-H), 6.53 (s, 1H, 8-H), 6.47 (dd, J = 1.7, 8.3 Hz, 1H 6"-H), 6.33 (s, 2H, 2',6'-H), 5.97 (dd, J = 1.2, 9.3 Hz, 2H, OCH₂O), 5.43 (s, 1H, exchangeable, 4'-OH), 4.65 (d, J = 4.2 Hz, 1H, 4-H), 4.60 (d, J = 4.8 Hz, 1H, 1-H), 4.39 (t, J = 7.3 Hz, 1H, 11-H) 3.96 (t, J = 7.3 Hz, 1H, 11-H), 3.90 (d, J = 6.2, Hz, 1H, exchangeable, NH), 3.80 (s, 6H, 3,'5'-OCH₃), 3.10 (dd, J = 4.9, 14.0 Hz, 1H, 2-H), 3.02 (m, 1H, 3-H). Anal. C₂₇H₂₄BrNO₇) C.H.N.

### EXAMPLE 40

### 4'-Demethyl-4β-[4"-bromoanilinyl]-4-desoxypodophyllotoxin.

Crystals from ethyl acetate/ethanol; mp 227-230°C;[α]²⁵D-110° (c = 0.5, CHCl₃); IR (KBr) 3500 (OH), 3330 (NH), 2900 (aliphatic CH), 1755 (lactone), 1605, 1590 and 1475 (aromatic C=C) cm⁻¹; ¹H NMR (CDCl₃) δ 7.30 (d, J = 8.9, Hz, 2H, 3",5"-H), 6.75 (s, 1H, 5-H), 6.53 (s, 1H, 8-H), 6.44 (d, J = 8.9 Hz, 2H, 2",6"-H), 6.32 (s, 2H, 2',6'-H), 5.98 (ABq, J = 1.3, 8.3 Hz, 2H, OCH₂O), 5.42 (s, 1H, exchangeable, 4'-OH), 4.62 (m, 2H, 4-H and 1-H), 4.36 (t, J = 8.5 Hz, 1H, 11-H), 3.95 (t, 8.5 Hz, 11-H), 3.86 (d, J = 7.8, 1H, exchangeable, NH), 3.79 (s, 6H, 3,'5'-OCH₃), 3.11 (dd, J = 4.8, 14.1 Hz, 1H, 2-H), 3.00 (m, 1H, 3-H). Anal. C₂₇H₂₄BrNO₇) C.H.N.

### EXAMPLE 41

### 4'-Demethyl-4β-[4"-iodoanilinyl]-4-desoxypodophyllotoxin.

Crystals from ethanol; mp 198-200°C(dec.);[α]²⁵D-111° (c = 0.5, CHCl₃); IR (KBr) 3540 (OH), 3420 (NH), 2900 (aliphatic CH), 1770 (lactone), 1610, 1585 and 1480 (aromatic C=C) cm⁻¹; ¹H NMR (CDCl₃) δ 7.46 (d, J = 8.8 Hz, 2H, 2",6"-H), 6.32 (s, 2H, 2',6'-H), 5.59 (ABq, J = 1.1, 8.9 Hz, 2H, OCH₂O), 5.44 (s, 1H, exchangeable, 4'-OH), 4.62 (m, 1H, 4-H), 4.58 (d, J = 4.9 Hz, 1H, 1-H), 4.34 (t, J = 8.5 Hz, 1H, 11-H), 3.94 (m, 2H, 11-H and NH), 3.78 (s, 6H, 3',5'-OCH₃), 3.09 (dd, J = 4.9, 14.1 Hz, 1H, 2-H), 2.99 (m, 1H, 3-H). Anal. C₂₇H₂₄BrNO₇) C.H.N.

### EXAMPLE 42 - 44 (Scheme IX)

Podophyllotoxin (500mg, 1.2 mmol) was dissolved in dry dichloromethane (10ml) and kept at 0°C. Hydrogen bromide gas was introduced into the solution for 45 min., after which time the solvent was evaporated in vacuo, anhydrous tetrahydrofuran (15 ml), anhydrous barium carbonate (474 mg, 2.4 mmol) and the appropriate hydroxyaniline (142mg, 1.3 mmol) was added. The mixture stood at room temperature overnight, and then was filtered and concentrated. The crude product was purified using column chromatography (silica gel 45 g with dichloromethane-acetone-ethyl acetate 100:5:5 as an eluant). The products (42-44) obtained in the examples had the characteristics listed below.

### EXAMPLE 42

### 4β-[2"-hydroxyanilinyl]-4-desoxypodophyllotoxin.

Amorphous crystals from ether: mp 145-148°C; IR (KBr) 3480 (OH), 3410 (NH), 2900 (aliphatic CH), 1760 (lactone), 1580, 1475 (aromatic C=C) cm⁻¹; H NMR (CDCl₃) δ 6.90 (t,J = 6.6 Hz, 1H, 4"-H), 6.78 (s, 1H, 5-H), 6.65 (m, 2H, 3",6"-H), 6.53 (m, 2H,8-H, 5"-H), 6.34 (s, 2H 2',6'H), 5.96 (ABq, J = 1.0, 3.5 Hz,2H, OCH₂O), 5.02 (s, 1H, exchangeable, 2"-OH), 4.68 (m, 1H, 4-H), 4.62 (d, J = 4.9 Hz, 1H, 1-H), 4.38 (t, J = 8.6 Hz, 1H, 11-H), 4.33 (m, 1H, exchangeable, NH), 4.00 (t, J = 8.6 Hz, 1H, 11H), 3.82 (s, 3H, 4'-OCH₃), 3.76 (s, 6H, 3',5'-OCH₃), 3.25 (dd, J = 5.1, 14.0 Hz, 1H, 2-H), 3.05 (m, 1H, 3-H). MS, m/z = 505 (m+). Anal. (C₂₈H₂₇NO₈ 3/2 H₂O) C.H.

### EXAMPLE 43

### 4β-[3"-hydroxyanilinyl]-4-Desoxypodophyllotoxin.

Amorphous powder from ether, mp 148-150°C; IR(KBr) 3370 (OH,NH), 2900 (aliphatic CH), 1760 (lactone), 1585, 1475 (aromatic C=C) cm⁻¹; ¹H NMR (CDCl₃) δ 7.05 (t, J = 8.0 Hz, 1H, 5"-H), 6.77 (s, 1H, 5H), 6.52 (s, 1H, 8-H), 6.32 (s, 2H, 2' 6'-H), 6.25 (dd, J = 2.2, 8.0 Hz, 1H, 4"-H), 6.14 (dd, J = 2.2, 8.0 Hz, 1H, 6"-H), 6.05 (t = 2.2 Hz, 1H, 2"-H), 5.96 (ABq, J = 1.3, 3.8 Hz, 2H OCH₂O), 4.64 (d, J = 3.9 1H, 4-H), 4.49 (d, J = 5.0, Hz, 1H, 1-H), 4.4 (t, J = 8.7 Hz, 1H, 11-H), 4.03 (t, J = 8.7 Hz, 1H, 11-H), 3.81 (s, 3H, 4'OCH₃), 3.76 (s, 6H, 3',5'-OCH₃), 3.18 (s, 6H, 3',5'-OCH₃), 3.18 (dd, J = 5.0 Hz, 14.0 Hz, 1H,2-H), 3.02 (m, 1H, 3-H); MS, m/z = 505 (m+). Anal. (C₂₈H₂₇NO₈ H₂O) C.H.

### EXAMPLE 44

### 4β-[4"-hydroxyanilinyl]-4-desoxypodophyllotoxin.

Crystals from chloroform; mp 145-150°C; IR(KBr) 3310 (OH,NH), 3010 (aromatic CH), 2900 (aliphatic CH), 1730 (latone), 1575, 1475 (aromatic C=H) cm⁻¹; ¹H NMR (CDCl₃) D₂O exchange) δ 6.75 (d, J = 8.3 Hz, 3H, 5-H, 3",5"-H), 6.53 (s, 1H, 8H), 6.45 (d, J = 8.3 Hz, 2H, 2",6"-H), 6.23 (s, 2H 2'6'-H), 5.95 (ABq, J = 1.0, 4.0 Hz, 2H OCH₂O), 4.60 (d, J = 4.2 Hz, 1H, 4-H), 4.57 (d, J = 4.6 Hz, 1H, 1-H), 4.38 (t, J = 6.0 Hz, 1H, 11-H), 4.05 (t, J = 6.0 Hz, 1H, 11H) 3.83 (s, 3H, 4-OCH₃), 3.75 (s, 6H, 3',5'-OCH₃), 3.18 (dd, J = 4.6 Hz, 14.0 Hz, 1H,2-H), 3.0 (m, 1H, 3-H).
Anal. (C₂₈H₂₇NO₈ 1/2 H₂O) C.H.

### Isolation of Human DNA Topoisomerase II.

Human DNA topoisomerase II was isolated from peripheral blast cells of a patient with acute leukemia. The isolation procedure is described in Thurston, L., Imakura, Y., Haruna, M., Li, Z. C., Liu, S. Y., and Lee, K. H., J. Med. Chem., 31, COMPLETE (1988) and is a partial combination of the procedure described in Goto, T., Laiapia, P. and Wang, J., J. Biol. Chem., 259, 10422 (1984) and Halligan, B., Edwards, K., and Liu, L., J. Biol. Chem., 260, 2475 (1985) which are herein specifically incorporated by reference.

### Preparations of Drugs.

Drugs were dissolved in Me₂SO at a concentration of 20 mM as the stock solution and diluted before use with water to the desired concentration of each drug.

### DNA Topoisomerase II Assay.

The P4 unknotting reaction was a modification of the procedure described by Hseih, T., J. Biol. Chem., 258, 8413 (1985), which is herein specifically incorporated by reference.

The reaction mixture (20 µL), which contained 50 mM HEPES, pH 7.0, 50 mM KCI, 100 mM NaCl, 0.1 mM EDTA, 10 mM MgCl₂, 1.0 mM ATP, 50 µg/mL bovine serum albumin, 0.4 µg P4 knotted DNA, and enzyme, was incubated with or without drugs.

The reaction mixture was incubated at 37° C for 30 min and terminated by adding 5.0 µl of a stop solution (2% sodium dodecyl sulfate, 20% glycerol, 0.05% bromophenol blue). These samples were loaded onto a 1% agarose gel and electrophoresed at 55 V overnight with an electrophoresis buffer that contained 90 mM Tris-boric acid, pH 8.3, and 2.5 mM EDTA. At completion, the gel was stained in 0.5 µg/mL of ethidium bromide. Then a photograph was taken of the DNA bands visualized with fluorescence induced by a long-wavelength UV lamp. The data reported in Table 1 reflect a 100 µM drug concentration.

### K-SDS Precipitation Assay for Protein-DNA Complexes.

The intracellular formation of covalent topoisomerase II-DNA complexes was quantitated using the potassium SDS precipitation assay, a procedure adapted from the method described in Rowe, T.C., Chen, G. L., Hsiang, Y. H., and Liu, L., Cancer Res., 46, 2021 (1986) (hereinafter Rowe et al.), which is herein specifically incorporated by reference. KB ATCC cells were prelabeled with 0.05 mCi/ml ¹⁴C-thymidine (specific activity 50.5 mCi/mmol) for 18 hr. A final concentration of 5x10⁵ cells/sample were treated with 10 µM of the drugs at 37° C for 1 hr and proceeded according to the procedure described by Rowe et al. to detect the protein-linked DNA levels.

It will be apparent to those skilled in the art that various modifications and variations can be made in the processes and products of the present invention. Thus, it is intended that the present invention cover the modifications and variations of this invention provided that they come within the scope of the appended claims.

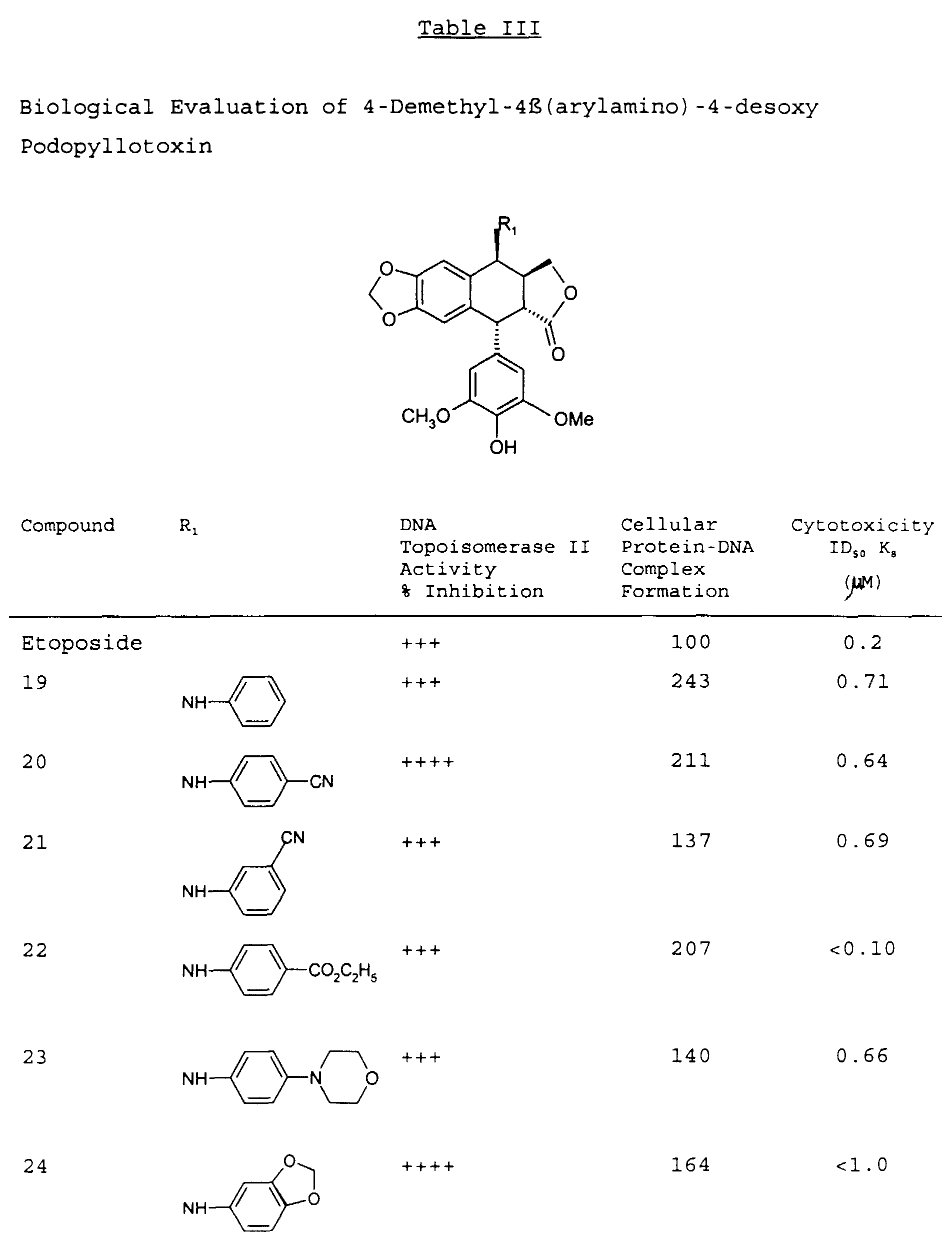

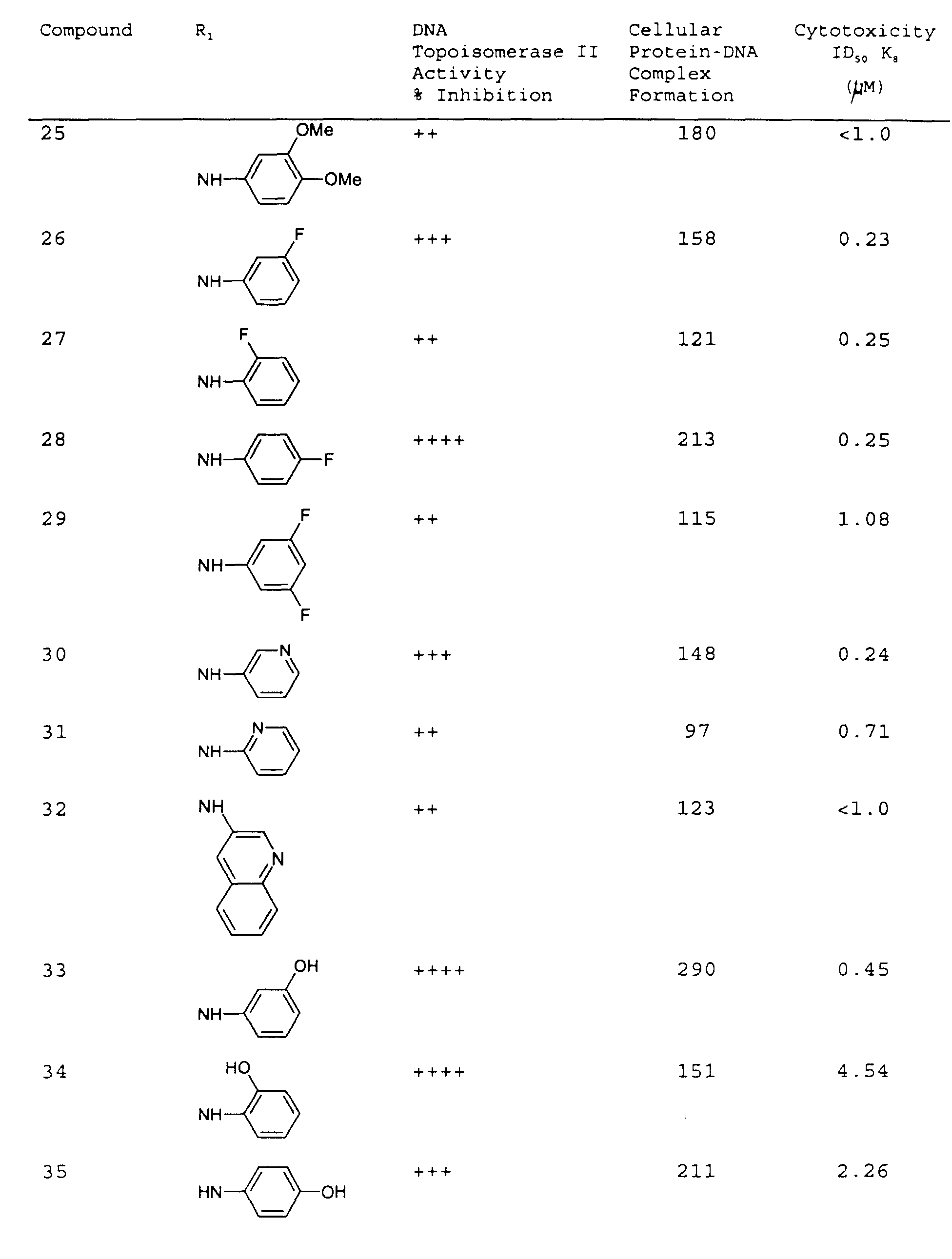

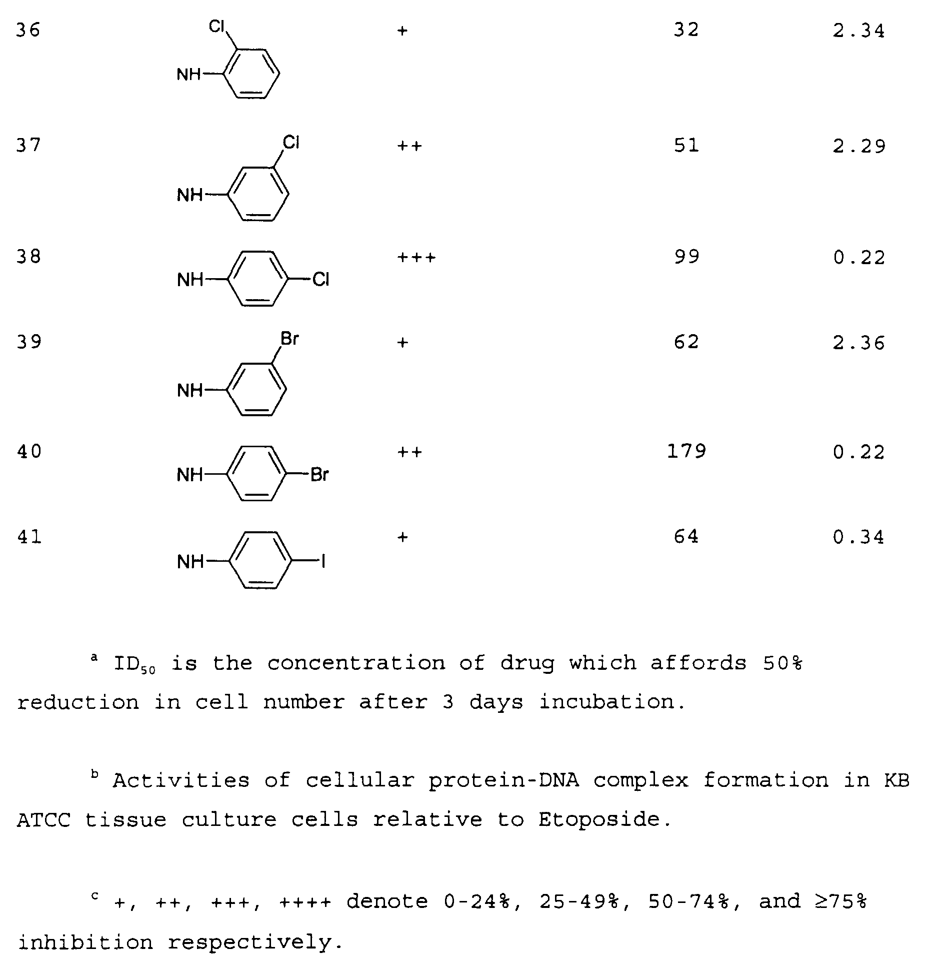

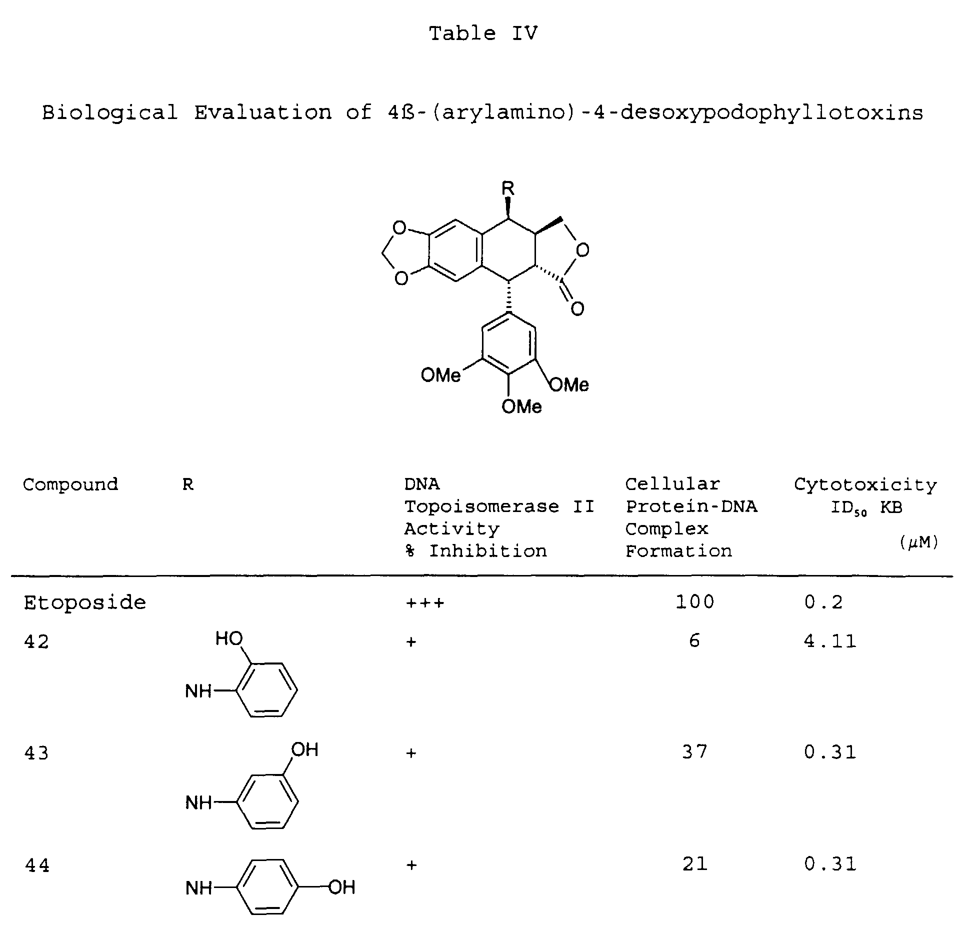

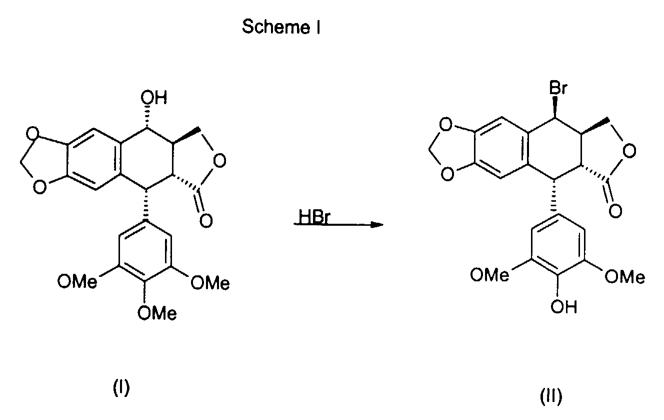

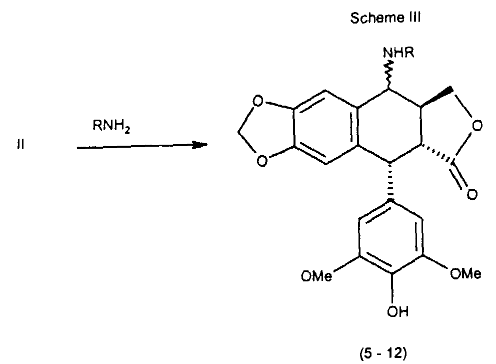

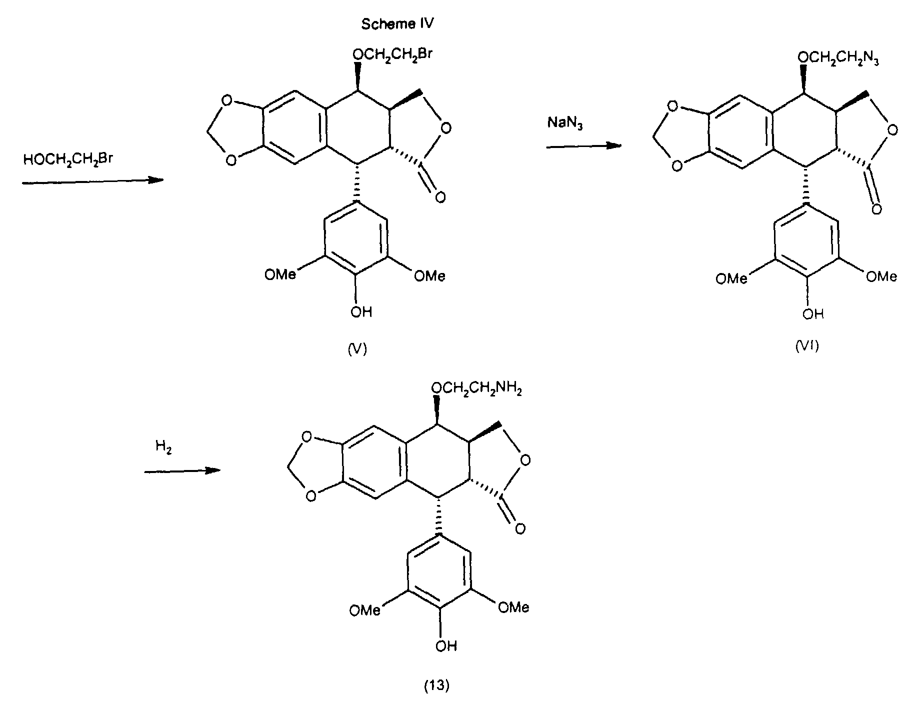

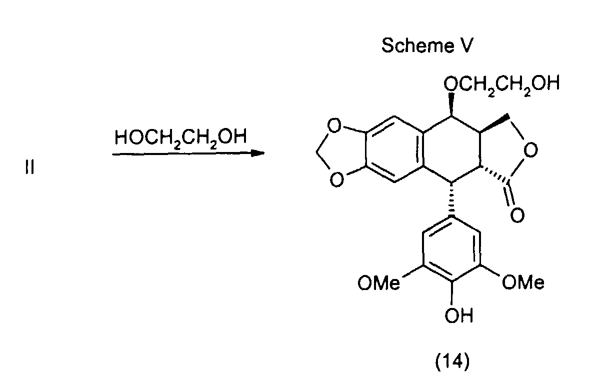

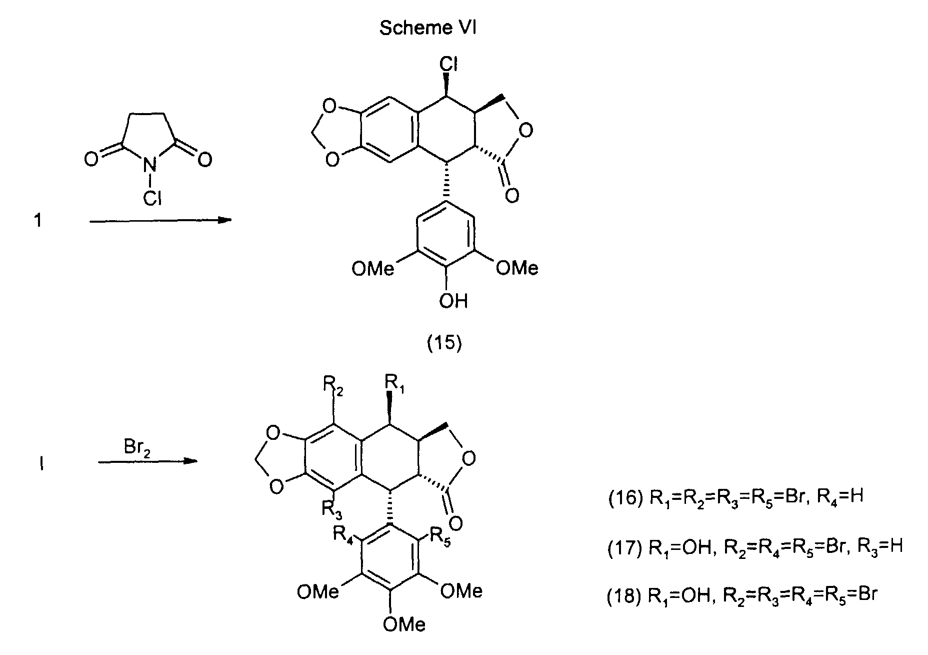

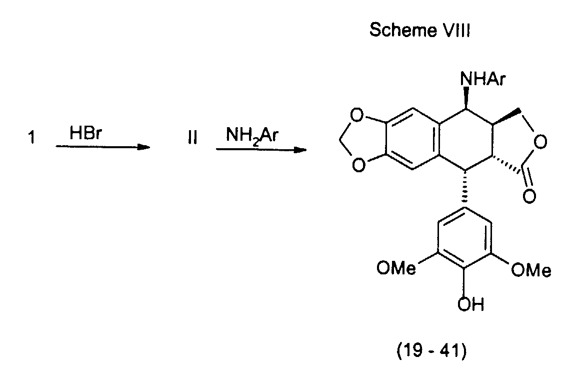

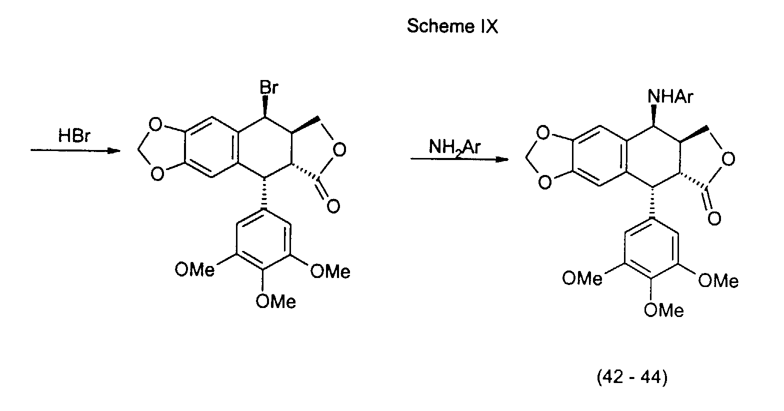

## Claims

1. A compound having the formula: 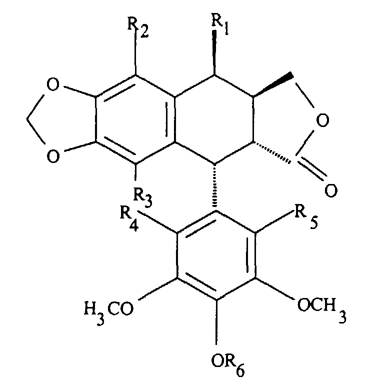 wherein
R₁ is 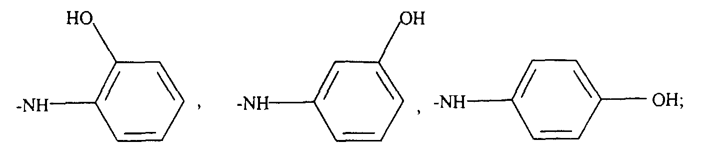
R₂ is H, or Br;
R₃ is H, or Br;
R₄ is H, or Br;
R₅ is H, or Br; and
R₆ is H, or -CH₃.

2. A compound as claimed in Claim 1, wherein R₂ to R₆ are H.

3. A compound of the formula 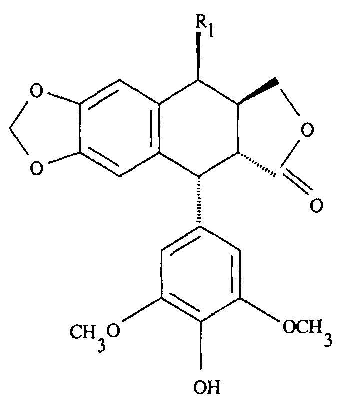 wherein R₁ is:

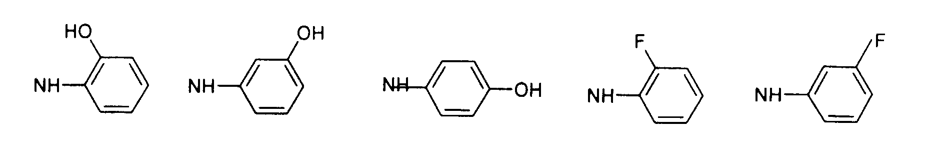

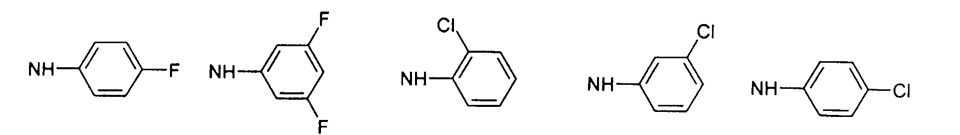

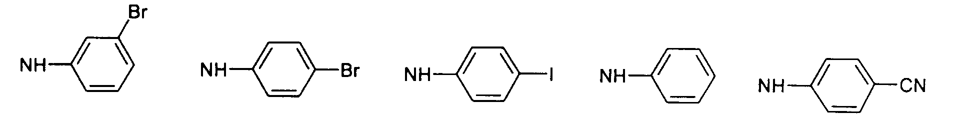

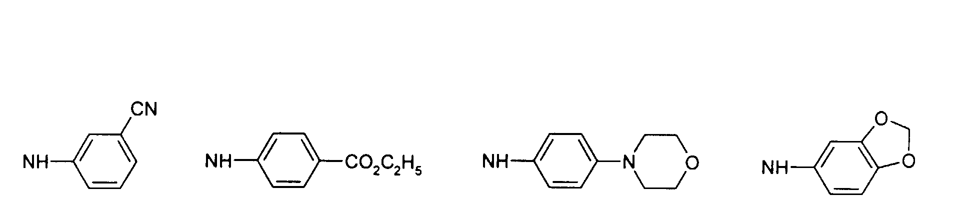

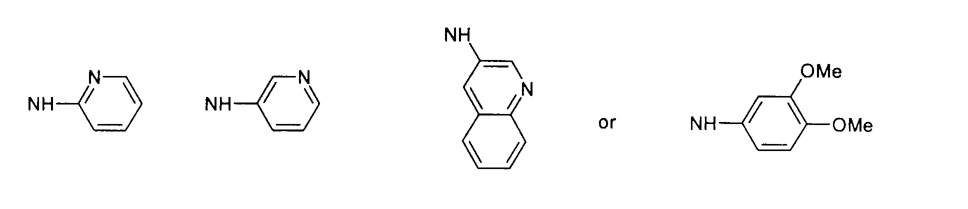

4. A compound of the formula 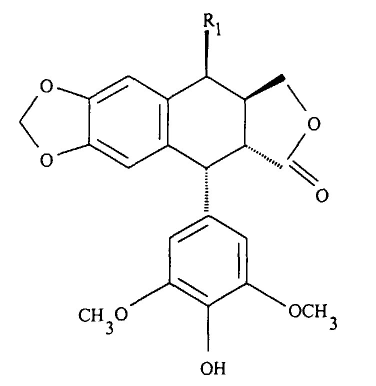 wherein R₁ is an arylamine of formula: 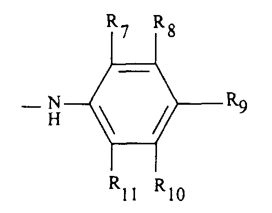 wherein
R₇, is H, OH, F, Cl, Br, CO₂CH₃, CO₂C₂H₅, CN, NO₂, NH₂, N(CH₃)₂, OCH₃, CH₂OH, CH₃, CF₃, CH₂CH₂OH, COCH₃, CH₂NH₂;
R₈ is H, OH, F, Cl, Br, CO₂CH₃, CO₂C₂H₅, CN, NO₂, NH₂, N(CH₃)₂, OCH₃, CH₂OH, CH₃, CF₃, CH₂CH₂OH, COCH₃, CH₂NH₂, CHOHCH₃, SCH₃;
R₉ is H, OH, F, Cl, Br, I, CHOCH₃, CO₂C₂H₅, CN, NO₂, NH₂, N(CH₃)₂, OCH₃, CH₂OH, CH₃, CF₃, CH₂CH₂OH, COCH₃, CH₂NH₂, N(CH₂CH₂OH)₂, 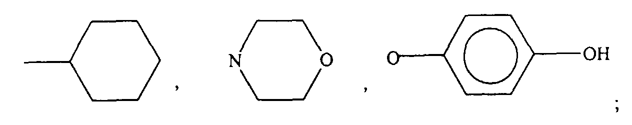
R₁₀ is H, F, Cl, OH, OCH₃, CO₂CH₃, CO₂C₂H₅, CH₃, CF₃, NO₂, NH₂, Cl;
R₁₁ is H, F, Cl, CH₃, CF₃, OH, OCH₃, NO₂;
R₉ and R₁₀ taken together are OCH₂O or OCH₂CH₂O.

5. A pharmaceutical composition comprising a compound as claimed in any one of the preceding claims.

## Patentansprüche

1. Verbindung der Formel: 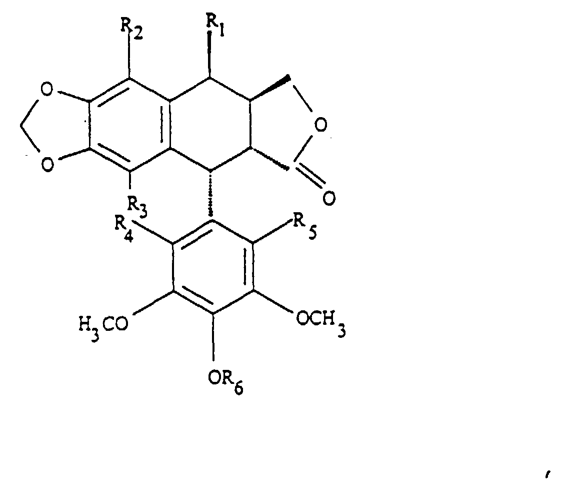 in der
R₁ 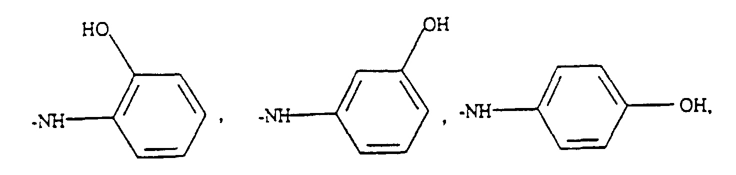
R₂ H oder Br,
R₃ H oder Br,
R₄ H oder Br,
R₅ H oder Br und
R₆ H oder -CH₃ ist.

2. Verbindung nach Anspruch 1, in der R₂ bis R₆ H sind.

3. Verbindung der Formel: 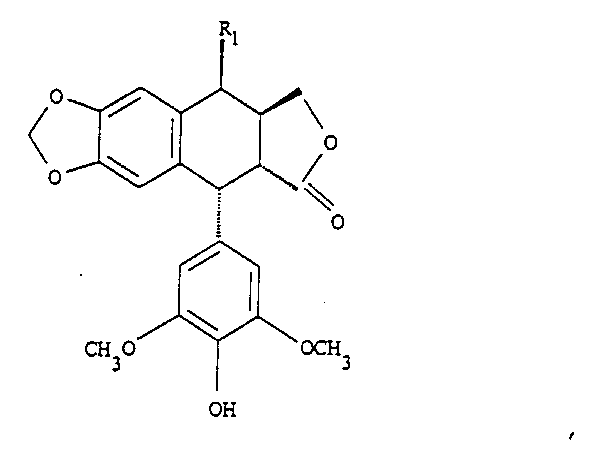 in der R₁: 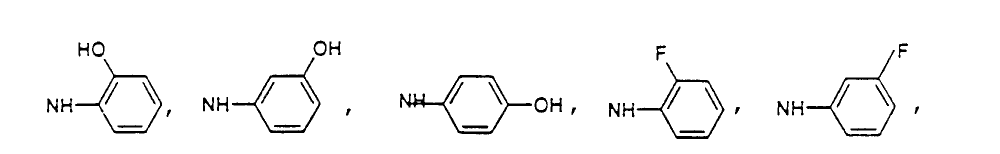 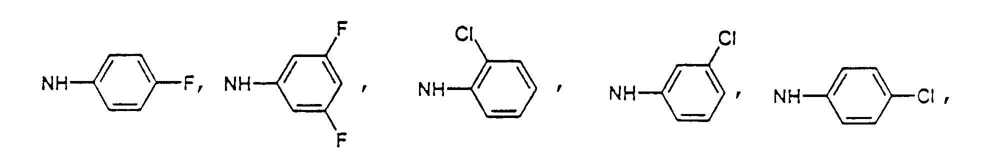 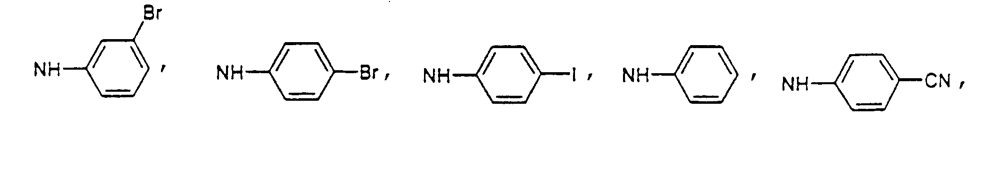 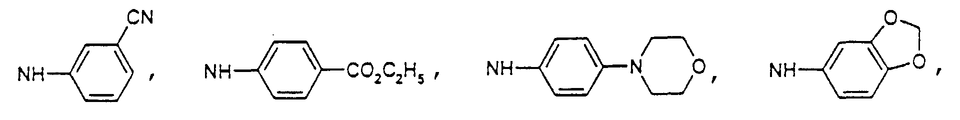 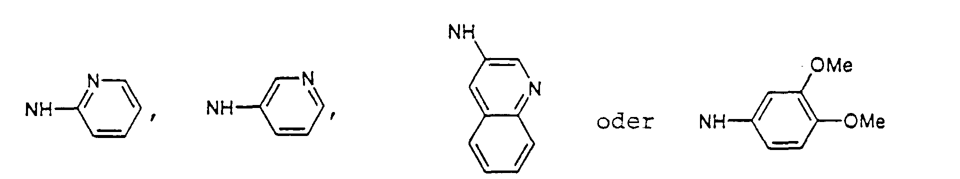 ist.

4. Verbindung der Formel 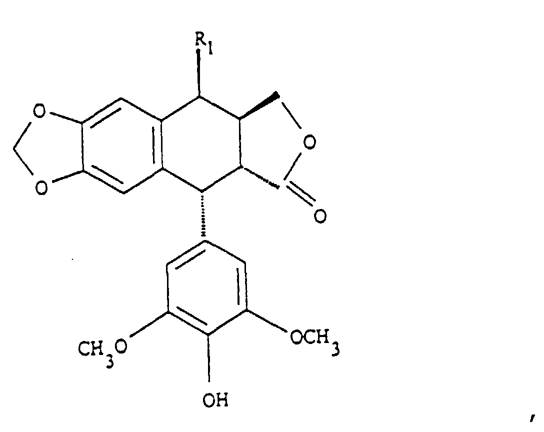 in der R₁ ein Arylamin der Formel: 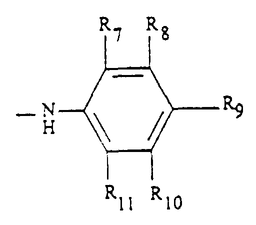 ist, in der
R₇ H, OH, F, Cl, Br, CO₂CH₃, CO₂C₂H₅, CN, NO₂, NH₂, N(CH₃)₂, OCH₃, CH₂OH, CH₃, CF₃, CH₂CH₂OH, COCH₃, CH₂NH₂,
R₈ H, OH, F, Cl, Br, CO₂CH₃, CO₂C₂H₅, CN, NO₂, NH₂, N(CH₃)₂, OCH₃, CH₂OH, CH₃, CF₃, CH₂CH₂OH, COCH₃, CH₂NH₂, CHOHCH₃, SCH₃,
R₉ H, OH, F, Cl, Br, I, CHOCH₃, CO₂C₂H₅, CN, NO₂, NH₂, N(CH₃)₂, OCH₃, CH₂OH, CH₃, CF₃, CH₂CH₂OH, COCH₃, CH₂NH₂, N(CH₂CH₂OH)₂, 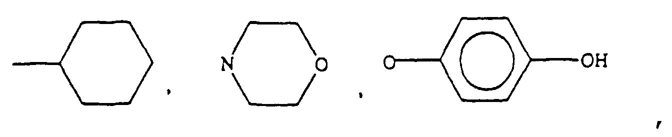
R₁₀ H, F, Cl, OH, OCH₃, CO₂CH₃, CO₂C₂H₅, CH₃, CF₃, NO₂, NH₂, Cl und
R₁₁ H, F, Cl, CH₃, CF₃, OH, OCH₃, NO₂ ist oder
R₉ und R₁₀ OCH₂O oder OCH₂CH₂O sind.

5. Pharmazeutische Zusammensetzung, die eine Verbindung gemäß einem der vorhergehenden Ansprüche enthält.

## Revendications

1. Composé ayant pour formule : 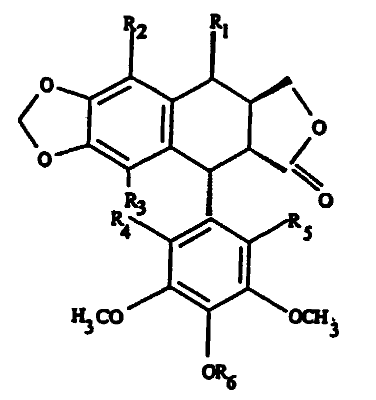 dans laquelle
R₁ est 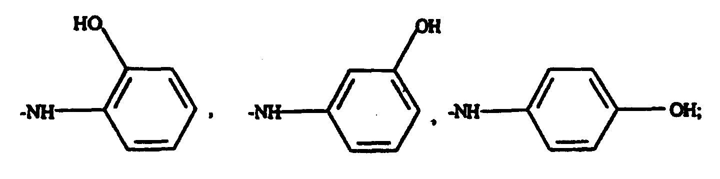
R₂ est H, ou Br ;
R₃ est H, ou Br ;
R₄ est H, ou Br ;
R₅ est H, ou Br ; et
R₆ est H, ou -CH₃.

2. Composé selon la revendication 1, dans lequel R₂ à R₆ sont H.

3. Composé de formule 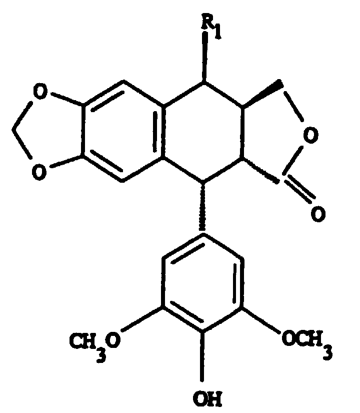 dans laquelle R₁ est :

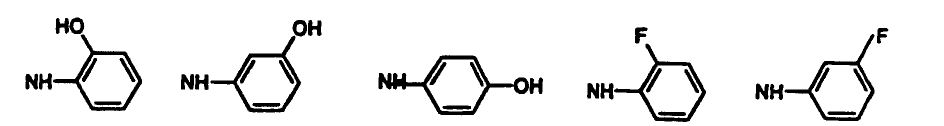

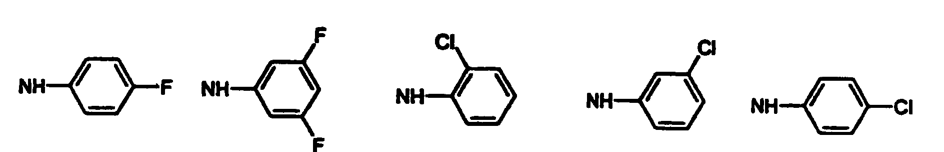

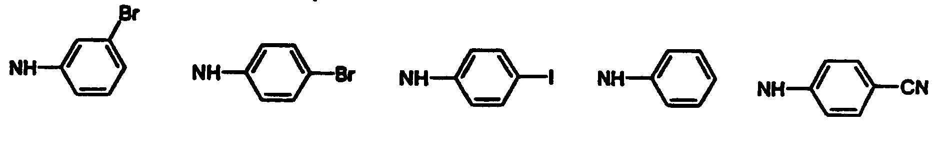

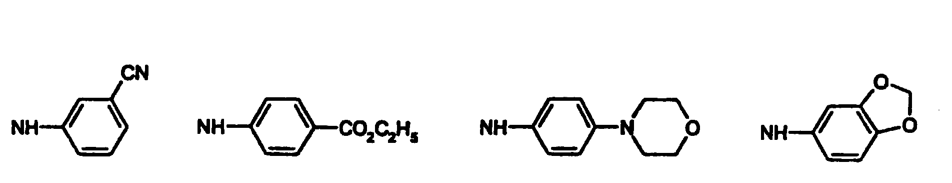

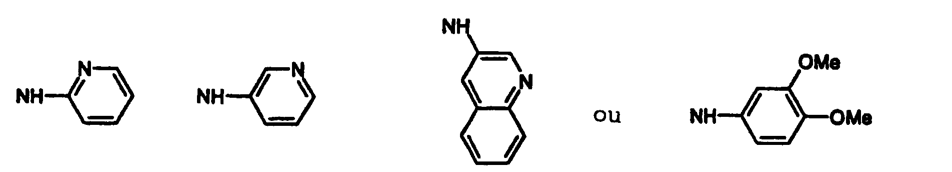

4. Composé de formule 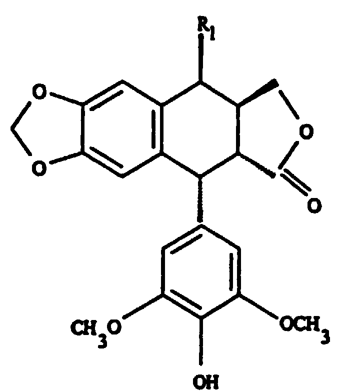 dans laquelle R₁ est une arylamine de formule : 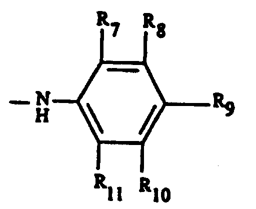 dans laquelle
R₇, est H, OH, F, Cl, Br, CO₂CH₃, CO₂C₂H₅, CN, NO₂, NH₂, N(CH₃)₂, OCH₃, CH₂OH, CH₃, CF₃, CH₂CH₂OH, COCH₃, CH₂NH₂;
R₈ est H, OH, F, Cl, Br, CO₂CH₃, CO₂C₂H₅, CN, NO₂, NH₂, N(CH₃)2, OCH₃, CH₂OH, CH₃, CF₃, CH₂CH₂OH, COCH₃, CH₂NH₂, CHOHCH₃, SCH₃ ;
R₉ est H, OH, F, Cl, Br, I, CHOCH₃, CO₂C₂H₅, CN, NO₂, NH₂, N(CH₃)₂, OCH₃, CH₂OH, CH₃, CF₃, CH₂CH₂OH, COCH₃, CH₂NH₂, N(CH₂CH₂OH)₂, 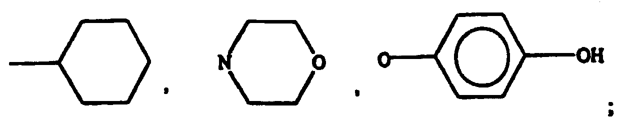
R₁₀ est H, F, Cl, OH, OCH₃, CO₂CH₃, CO₂C₂H₅, CH₃, CF₃, NO₂, NH₂, Cl ;
R₁₁ est H, F, Cl, CH₃, CF₃, OH, OCH₃, NO₂ ;
R₉ et R₁₀ pris ensemble sont OCH₂O ou OCH₂CH₂O.

5. Composition pharmaceutique comprenant un composé selon l'une des revendications précédentes.
